# EUROPEAN PATENT APPLICATION

(11) **EP 3 366 274 A1**
(43) Date of publication of application: **29.08.2018**
(21) Application number: 17207153.2
(22) Date of filing: 13.09.2013
(51) Int. Cl.: A61K 8/49, A61Q 19/08, A61K 8/19, A61N 5/06

(54) **COSMETIC BIOPHOTONIC COMPOSITIONS**

(30) Priority: 14.09.2012 US 201261701519 P; 14.03.2013 US 201361786084 P; 04.09.2013 US 201361873752 P
(62) Divisional of application: 16192956.7
(71) Applicant: KLOX Technologies, Inc., Laval QC H7V 4A7 (CA)
(72) Inventor: Loupis, Nikolaos, GR-14562 Athens (GR); Piergallini, Remigio, I-63038 Grottammare (IT); HÉBERT, Lise, Québec, H7V 4A7 (CA)
(74) Representative: Therias, Philippe

(57) **Abstract**

The present disclosure provides cosmetic compositions and methods for using the cosmetic compositions. In particular, the cosmetic compositions of the present disclosure include one or more chromophore(s) in association with a dermatologically acceptable carrier. The cosmetic biophotonic compositions and the methods of the present disclosure are useful for skin rejuvenation and/or skin conditioning.

## Description

### BACKGROUND OF THE DISCLOSURE

Visible signs of intrinsic and extrinsic ageing of skin include development of fine lines (wrinkles), loss of elasticity (sagging) as well as changes in skin tone, texture, thickness and moisture content. Cosmetic methods such as ablative and non-ablative skin rejuvenation, and low level light therapy (phototherapy) attempt to reverse the visible signs of ageing. Phototherapy is also used to control or treat skin conditions such as acne, psoriasis, rosacea and scarring.

In ablative skin rejuvenation, upper layers of skin are removed by lasers or chemical peels in order to induce regeneration of the skin. This is a painful, invasive procedure, with a prolonged recovery period and can have serious complications such as infection and scarring. Non-ablative skin rejuvenation methods on the other hand deliver thermal damage to the deeper layers of the skin whilst cooling the upper layers to protect them from thermal damage. Although less invasive than ablative methods, the process must also be performed in a clinic, is not without risks, is expensive and involves down-time for the patient. In low level light therapy (phototherapy) the skin is exposed to low level LED or laser light which poses lower risks compared to ablative and non-ablative processes. Home devices do exist, however, they are expensive, and there is down-time for the user during the treatment period.

Furthermore, the effects of the above cosmetic methods are often not permanent, necessitating repeat treatments despite efforts to maintain skin condition and to prevent or minimize ageing using for example cosmetic creams.

Therefore, it is an object of the present disclosure to provide a composition which can be used as a cosmetic composition and which can help to rejuvenate skin and/or which can maintain skin condition, for example after a cosmetic or a medical treatment.

### SUMMARY OF THE DISCLOSURE

The present disclosure generally relates to compositions and methods for rejuvenating skin and/or for maintaining and/or improving skin condition, said compositions and methods comprising one or more photoexcitable chromophores.

From one aspect there is provided a composition for cosmetic use comprising: a first chromophore in a dermatologically acceptable carrier; wherein the chromophore is photoactive in the dermatologically acceptable carrier and can absorb light from the visible portion of the electromagnetic spectrum

From another aspect there is provided a composition for cosmetic use comprising: a first chromophore in a dermatologically acceptable carrier; wherein the chromophore is photoactive in the dermatologically acceptable carrier and is present in a concentration which does not substantially colour the composition. In other words, the composition is substantially a neutral colour or a colour resembling a skin colour.

From yet another aspect there is provided a composition for cosmetic use comprising: a first chromophore in a dermatologically acceptable carrier; wherein the chromophore is photoactive in the dermatologically acceptable carrier and is present in a concentration such that photoactivation of the chromophore by ambient light or direct light does not cause the chromophore to emit fluorescence substantially visible to the human eye.

From a further aspect, there is provided a cosmetic composition for topical cosmetic use, the composition comprising: a first fluorescent chromophore and a dermatologically acceptable carrier; wherein the first chromophore is capable of absorbing and emitting light in the visible range of the electromagnetic spectrum and wherein the composition does not substantially visibly colour the skin after topical application of the composition to the skin in use.

From a yet further aspect, there is provided a cosmetic composition for topical cosmetic use, the composition comprising a first fluorescent chromophore, a second fluorescent chromophore, and a dermatologically acceptable carrier, wherein the first and second chromophores are capable of absorbing light in the visible range of the electromagnetic spectrum and have a total concentration by weight in the composition of about 0.001wt% to less than about 0.5wt%.

The disclosure contemplates that any of the embodiments set forth below can be combined with each other or with any of the aspects or embodiments set forth above, or otherwise set forth herein.

In certain embodiments, the dermatologically acceptable carrier is a cream, a lotion or a serum. The dermatologically acceptable carrier may be absorbed into the skin. In one embodiment, the dermatologically acceptable carrier is an emulsion. The dermatologically acceptable carrier may be aqueous.

In certain embodiments, the composition can be applied to skin and does not substantially visibly colour the skin after application. This can be achieved by, for example, providing an amount of the chromophore in the dermatologically acceptable carrier that is not sufficient to colour the skin, providing a chromophore that is not absorbed into live and/or dead cells in the skin, or including an agent in the composition to substantially counteract or minimize a colour effect on the skin of the first chromophore. In certain embodiments, molecules of the first chromophore can have a polarity such that it is not substantially absorbed by the skin. This means that the user can wear the composition on his or her face during at least the day. A slight fading of the colour of the composition may occur as the chromophore photobleaches.

In certain embodiments, the first chromophore is selected such that its photoexcitation by light can result in the subsequent emission of light (e.g. fluorescence or phosphorescence) and/or its transition to a singlet excited state and subsequent interaction with other molecules, for example molecular oxygen, to generate active oxygen (e.g. singlet oxygen). It is thought that low levels of active oxygen can have a beneficial effect on tissues such as skin by biomolecular signaling. It is also believed that low light intensities have a rejuvenating and therapeutic effect on tissues such as skin. This may increase collagen synthesis in the tissue and/or have an antimicrobial effect. Photoexcitation is preferably not accompanied by concomitant generation of heat. In other embodiments, the photoexcitation of the chromophore does not result in tissue damage.

In certain embodiments, the composition comprises a second chromophore, said second chromophore being photoexcitable in the composition. In certain embodiments, the second chromophore can absorb light in the visible range of the electromagnetic spectrum.

In certain embodiments, the first and/or the second chromophore (when present) can be photoexcited by ambient light including from the sun and/or direct light which can also include direct sunlight. In certain embodiments, the first and/or the second chromophore (when present) can be photoactivated by light in the visible range of the electromagnetic spectrum. The light can be emitted by any light source such as sunlight, light bulb, an LED device, electronic display screens such as on a television, computer, telephone, mobile device, flashlights on mobile devices. The first and/or the second chromophore (when present) may absorb light having a wavelength in the range of about 380-600 nm, about 400-500 nm, about 450-650 nm, about 600-700 nm, about 650-750 nm, about 400-750 nm, about 420-600nm, or about 450 to 550 nm.

The first chromophore, the second chromophore or both can be any chromophore or photoactive agent which can be excited by electromagnetic radiation to render a beneficial effect on skin. In certain embodiments, the first and/or the second chromophore (when present) is a fluorochrome and/or a xanthene derivative such as Eosin Y, Fluorescein, Rose Bengal, Erythrosine, Phloxine B. The first and/or the second chromophore (when present) can be a chlorophyll dye, such as chlorophyllin, chlorophyll a or chlorophyll b. The first and/or the second chromophores, or further chromophores included in the composition can be a methylene blue dye, an azo dye, or a naturally occurring photoactive dye.

In certain embodiments of any of the foregoing or following, the first chromophore of the cosmetic composition is present in an amount of 0.001-40% per weight of the composition, about 0.001-20%, about 0.001-10%, about 0.001-5%, about 0.001-1%, about 0.001-0.5%, 0.001-0.1%, or about 0.001-0.01%. The second chromophore, when present, is present in an amount of 0.0003-40%, about 0.0003-20%, about 0.0003-10%, about 0.0003-5%, or about 0.0003-1% per weight of the composition. In certain embodiments, the total weight per weight of chromophore or combination of chromophores may be in the amount of about 0.001-40.0003% per weight of the composition.

In certain embodiments where the first and second chromophores are present, the first or the second chromophore has an emission spectrum that overlaps at least 20% of an absorption spectrum of the other of the first and second chromophores. The first or the second chromophore has an emission spectrum that overlaps at least 5%, 10%, 20%, 25%, 30%, 40%, 50%, 60%, 70% of an absorption spectrum of the other of the first and second chromophores, when present. In some embodiments, the emission spectrum of first or the second chromophore overlaps at least 1-10%, 5-15%, 10-20%, 15-25%, 20-30%, 25-35%, 30-40%, 35-45%, 50-60%, 55-65% or 60-70% of the absorption spectrum of the other chromophore when present. Photoactivation of one of the first or the second chromophores may photoactivate the other of the first and second chromophores. The photoactivated second chromophore may modulate collagen synthesis, modulate inflammation or have an antimicrobial effect.

In certain embodiments, one of the first and second chromophores absorbs at an average peak wavelength that is relatively longer than that of the other of the first and second chromophores, and within the range of about 10-100 nm. In some embodiments, one of the first and second chromophores absorbs at an average peak wavelength that is relatively longer than that of the other of the first and second chromophores and within the range of about 20-80 nm, 20-60 nm or 20-50 nm. A transfer of energy may be obtained between the first and second chromophores resulting in photoactivation of both chromophores with the same light source.

In certain embodiments, when exposed to the ambient and/or direct light, the composition can emit light having a wavelength from about 480 nm to about 650 nm, about 500 nm to about 650 nm, about 520 nm to about 650 nm, or about 520 nm to about 600 nm.

In certain embodiments of any of the foregoing or following, the composition does not include a self-tanning agent. In certain embodiments of any of the foregoing or following, the composition does not include cleansers. In certain embodiments of any of the foregoing or following, the photoactivatable chromophore in the composition is not a chromophore which can absorb UV light. In certain embodiments of any of the foregoing or following, the chromophore is not insolubilized in the dermatologically accepted carrier, or rendered non-photoactive in any other way. At least one of the first and second fluorescent chromophores can be in a soluble form in the composition.

In certain embodiments of any of the foregoing or following, exposure of the composition to light, e.g., ambient light or direct light including from the sun, results in photoexcitation of at least one of the chromophores, and possibly an energy transfer between the chromophores. In one embodiment, the cascade of energy transfer provides photons that penetrate the epidermis and/or dermis at the target tissue site (e.g. skin). In another embodiment, there is a reaction with molecular oxygen to generate active oxygen species (e.g. singlet oxygen). In another embodiment, the cascade of energy transfer is not accompanied by concomitant generation of heat. In yet another embodiment, the cascade of energy transfer does not result in tissue damage.

In certain embodiments, the composition does colour the skin, for example to enhance or even out the user's natural skin colour or tone, or to cover blemishes on the skin, such as in a tinted moisturizer or foundation. In this case, the colour may come from the first or the second chromophore (when present), or from a colouring agent which may or may not be photoexcitable.

The first and the second chromophores (when present) may at least partially photobleach (loss of colour) with exposure to electromagnetic radiation such as light. Depending on the strength of the starting colour of the composition, this may provide the user with a stronger colour when the composition is first applied to the skin, and fade gradually on exposure to light. Advantageously, this can compensate a user's natural pallor first thing in the morning, and fade as the user's colour naturally increases as the day wears on. The rate of photobleaching may be equated with the rate of decrease of the power density of the fluorescent light irradiating the skin.

In certain embodiments of any of the foregoing or following, the composition optionally includes other cosmetic, dermatological and pharmaceutical active agents such as collagen synthesis promoting agents, healing factors, antimicrobial and antifungal agents, anti-viral agents, anti-wrinkle and skin repair agents, skin barrier repair agents, anti-inflammatory agents, skin conditioners, preservatives, and sunscreen agents.

From another aspect, there is provided a method for cosmetically rejuvenating skin or maintaining skin condition comprising: topically applying a composition of the disclosure, as described herein, onto a skin area, the composition comprising a first chromophore in a dermatologically acceptable carrier; and illuminating said composition with ambient light and/or direct light containing a wavelength that can be absorbed by the first chromophore; wherein the chromophore is photoactive in the dermatologically acceptable carrier and can absorb light from the visible portion of the electromagnetic spectrum.

From yet another aspect, there is provided a method for rejuvenating skin or maintaining skin condition comprising: topically applying a composition of the disclosure, as described herein, onto a skin area, the composition comprising a first chromophore in a dermatologically acceptable carrier; and illuminating said composition with ambient light and/or direct light containing a wavelength that can be absorbed by the first chromophore; wherein the chromophore is photoactive in the dermatologically acceptable carrier and is present in a concentration which does not substantially colour the composition.

From a further aspect, there is provided a method for rejuvenating skin or maintaining skin condition comprising: topically applying a composition of the disclosure, as described herein, onto a skin area, the composition comprising a first chromophore in a dermatologically acceptable carrier; and illuminating said composition with ambient light and/or direct light containing a wavelength that can be absorbed by the first chromophore; wherein the chromophore is photoactive in the dermatologically acceptable carrier and is present in a concentration such that photoactivation of the chromophore by ambient or direct light does not cause the chromophore to emit fluorescence substantially visible to the human eye.

From a yet further aspect, there is provided a method for rejuvenating skin or maintaining skin condition comprising: topically applying a composition onto a skin area, the composition comprising a first fluorescent chromophore, a second fluorescent chromophore, and a dermatologically acceptable carrier, wherein the first and second chromophores are capable of absorbing light in the visible range of the electromagnetic spectrum and have a total concentration by weight in the composition of about 0.001wt% to less than about 0.5wt%; and - illuminating said composition with ambient light and/or direct light containing a wavelength that can be absorbed by the first fluorescent chromophore.

From another aspect, there is provided a method for rejuvenating skin or maintaining skin condition comprising: topically applying a composition onto a skin area, the composition comprising a first fluorescent chromophore and a dermatologically acceptable carrier, wherein the first fluorescent chromophore is capable of absorbing light in the visible range of the electromagnetic spectrum, and emits light having a wavelength between about 480 nm to about 650 nm; and illuminating said composition with ambient light and/or direct light containing a wavelength that can be absorbed by the first fluorescent chromophore.

There is also provided a method for rejuvenating skin or maintaining skin condition, the method comprising illuminating the skin with an emitted light from an activated fluorescent chromophore, wherein the emitted light has a wavelength of about 480 nm to about 650 nm. In certain embodiments, the fluorescent chromophore is activated by white light. In certain embodiments, the power density of the emitted light decreases over time. Activation by white light can provide a slower rate of power density decrease of the emitted light compared to a single peak activating light.

There is also provided a method for rejuvenating skin, maintaining skin condition and/or for stimulating/increasing collagen formation comprising illuminating skin using an overlap in an emission spectra of a first and second fluorescent chromophore to emit light having a wavelength of about 480 nm to about 650 nm.

From another aspect there is also provided a method of using a cascade of energy transfer between at least a first and a second fluorescent chromophore to absorb and/or emit light within the visible range of the electromagnetic spectrum for rejuvenating skin or maintaining skin condition.

In certain embodiments, the composition is illuminated for a total of at least 30 minutes by an ambient light or by direct light including sunlight. Depending on the time of day and the season, 13 minutes of sunlight may be enough to substantially photoexcite the chromophore(s). In certain embodiments, the composition is illuminated directly for a total of at least 15 minutes by an artificial light source such as a lamp, a mobile device or a display screen.

In some embodiments, the composition may be applied once during the day in the morning. In this case, depending on the activities of the user and the light exposure, the chromophore may remain photoactive during the course of the day, for example over a period of about 30 minutes, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, or about 18 hours.

According to other embodiments of the methods of the present disclosure, a pre-conditioning or post-conditioning composition may be applied to the target tissue, before, during or after the application of the presently disclosed composition. The conditioning composition may be a serum including for example naturally derived protective compounds or free radical quenchers (e.g. hydroxyl radical quenchers and superoxide anion quenchers), which can be reactive in the presence of some chromophores and therefore advantageous to apply to the target site separately. The preconditioning composition may include an antibacterial or oxygen-releasing agent. It may be in the form of a wash or a soap.

In certain embodiments of any of the foregoing or following, the cosmetic composition and the method promotes skin rejuvenation, maintenance of skin condition and/or skin conditioning.

From another aspect, there is provided use of the composition as described herein for skin rejuvenation, skin conditioning and/or skin condition maintenance. One such use is daily to three times daily application of the present composition to maintain skin condition after an invasive skin rejuvenation procedure, or after treatment of a skin condition such as acne by light therapies or other treatments.

From another aspect, there is provided use of a mobile device to activate a photoactivatable composition, such as the composition described herein, wherein the mobile device can emit light having an emission spectra which overlaps an absorption spectra of a photoactive agent in the photoactivatable composition. The mobile device can have a display screen through which the light is emitted and/or the mobile device can emit light from a flashlight which can photoactivate the photoactivatable composition. For example, the wavelength and power density of the emitted light may be controlled by a computing device in the form of an app or program.

From a further aspect, there is provided use of a display screen on a television or a computer monitor to activate a photoactivatable composition as described herein, wherein the display screen can emit light having an emission spectra which overlaps an absorption spectra of a photoactive agent in the photoactivatable composition.

From a yet further aspect, there is provided use of an emitted fluorescent light from a composition as described herein to generate collagen in tissue to which the collagen is applied.

### BRIEF DESCRIPTION OF THE DRAWINGS

**Figure 1** illustrates the electronic states of a molecule and the transitions between them ('Jablonski diagram');
**Figure** 2 illustrates the difference in wavelength between positions of the band maxima of the absorption and emission spectra of the same electronic transmission ('Stokes' shift');
**Figure 3** illustrates absorption of light in the various layers of the skin (Samson et al. Evidence Report/Technology Assessment 2004, 111, pages 1-97); and
**Figure 4** illustrates the absorption and emission spectra of donor and acceptor chromophores, including the spectral overlap between the absorption spectrum of the acceptor chromophore and the emission spectrum of the donor chromophore.
**Figures 5A, 5B** **and** **5C** are emission spectra of one embodiment of the composition of the present disclosure after 3, 6 and 16.5 hours respectively of ambient light exposure (Example 1).
**Figure 6** is an emission spectrum of the composition of Figures 5a, 5b and 5c after 6 hours of direct light exposure (Example 1).
**Figures 7A, 7B** **and** **7C** illustrate emission spectra of another embodiment of the composition of the present disclosure after 3, 6 and 16.5 hours of ambient light exposure (Example 2).
**Figures 8A** and **8B** are absorbance and emission spectra, respectively, of a composition which includes Eosin and Fluorescein in a gel (Example 3).
**Figures 9A** and **9B** are absorbance and emission spectra, respectively, of a composition which includes Eosin and Fluorescein in an aqueous solution (Example 4).
**Figures 10A** and **10B** are absorbance and emission spectra, respectively, of a composition which includes Eosin Y and Phloxine **B** in a gel (Example 5)
**Figures 11A** and **11B** are absorbance and emission spectra, respectively, of a composition which includes Eosin Y and Phloxine B in aqueous solution (Example 6);
**Figures 12A** and **12B** are absorbance and emission spectra, respectively, of a composition which includes Eosin Y and Rose Bengal in a gel (Example 7);
**Figures 13A** and **13B** are absorbance and emission spectra, respectively, of a composition which includes Fluorescein and Phloxine B in a gel (Example 8).
**Figures 14A** and **14B** are absorbance and emission spectra, respectively, of a composition which includes Fluorescein and Rose Bengal in a gel (Example 9).
**Figures 15A** and **15B** are absorbance and emission spectra, respectively, of a composition which includes Eosin Y, Fluorescein and Rose Bengal in a gel (Example 10).
**Figures 16A and 16B** are absorbance and emission spectra, respectively, of a composition which includes Eosin Y, Fluorescein and Rose Bengal in an aqueous solution (Example 11).

### DETAILED DESCRIPTION

### (1) Overview

Skin is the largest organ of the body, accounting for 12% to 16% body weight. Skin is made up of two main layers that cover a third fatty layer. The outer layer is the epidermis, and second layer beneath epidermis is the dermis. Under these two skin layers is a fatty layer of subcutaneous tissue. With age the amount of subcutaneous (under-the-skin) fat is reduced resulting in a looser look to the skin. Skin changes, such as wrinkles and sagging skin, are among the most visible signs of aging.

The present disclosure provides compositions and methods which may be useful for skin rejuvenation and/or skin conditioning. In particular, the compositions of the present disclosure comprise a first chromophore in a dermatologically acceptable carrier; wherein the chromophore is photoexcitable in the dermatologically acceptable carrier. The compositions of the present disclosure may be considered to be cosmetic biophotonic compositions.

### (2) Definitions

Before continuing to describe the present disclosure in further detail, it is to be understood that this disclosure is not limited to specific compositions or process steps, as such may vary. It must be noted that, as used in this specification and the appended claims, the singular form "a", "an" and "the" include plural referents unless the context clearly dictates otherwise.

As used herein, the term "about" in the context of a given value or range refers to a value or range that is within 20%, preferably within 10%, and more preferably within 5% of the given value or range.

It is convenient to point out here that "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

"Ambient light" means light that fills an indoor space with illumination. The light can be from any source, such as the sun or artificial lighting.

"Direct light" means light irradiation directly from a light source, such as from a lamp, mobile device, screen, sun etc. In some cases, depending on the power density of the light emitted from the light source, direct light may be considered as ambient light at a far enough distance from that direct light.

"Biophotonic composition" is a composition as described herein that may be activated or excited by radiant energy to generate photons and/or chemical species, such as reactive oxygen species, including singlet oxygen, for therapeutic effect.

"Photoexcitable" or "photoactive" in relation to a chromophore is meant that the molecules of the chromophore are able to absorb radiant energy within the medium of the dermatologically acceptable carrier. The excited state is referred to herein, interchangeably, as 'photoexcited' or 'photoactivated'. A photoactivated chromophore may transition to an excited state leading to the emission of the absorbed energy as light, e.g. fluorescence, or to transfer of the absorbed energy to other molecules.

"Topical composition" means a composition to be applied to body surfaces, such as the skin, mucous membranes, vagina, oral cavity, lesions, scars, surgical wound sites, and the like. A topical composition may be in the form of, including, but not limited to, a cream, emulsion, gel, ointment, lotion, levigate, solution, paste, bioadhesive, salve, milk, impregnated pad, spray, suspension, foam, or the like.

Terms "chromophore", "photoactivating agent" and "photoactivator" are used herein interchangeably. A chromophore means a chemical compound, when contacted by light irradiation, is capable of absorbing the light. The chromophore can undergo photoexcitation and can then emit its energy as light or transfer its energy to other molecules to form oxygen species or free radicals.

"Skin rejuvenation" means a process of reducing, diminishing, retarding or reversing one or more signs of skin aging. For instance, common signs of skin aging include, but are not limited to, appearance of fine lines or wrinkles, thin and transparent skin, loss of underlying fat (leading to hollowed cheeks and eye sockets as well as noticeable loss of firmness on the hands and neck), bone loss (such that bones shrink away from the skin due to bone loss, which causes sagging skin), dry skin (which might itch), inability to sweat sufficiently to cool the skin, unwanted facial hair, freckles, age spots, spider veins, rough and leathery skin, fine wrinkles that disappear when stretched, loose skin, or a blotchy complexion. According to the present disclosure, one or more of the above signs of aging may be reduced, diminished, retarded or even reversed by the compositions and methods of the present disclosure.

Features and advantages of the subject matter hereof will become more apparent in light of the following detailed description of selected embodiments, as illustrated in the accompanying figures. As will be realized, the subject matter disclosed and claimed is capable of modifications in various respects, all without departing from the scope of the claims. Accordingly, the drawings and the description are to be regarded as illustrative in nature, and not as restrictive and the full scope of the subject matter is set forth in the claims.

### (3) Cosmetic Biophotonic Topical Compositions

The present disclosure provides cosmetic biophotonic compositions. Cosmetic biophotonic compositions are, in a broad sense, compositions that can be activated by light. These compositions contain at least one chromophore which can be excited by light to emit light of a different wavelength than the absorbed light, and/or to cause photochemical activation of other agents contained in the composition or in the tissue.

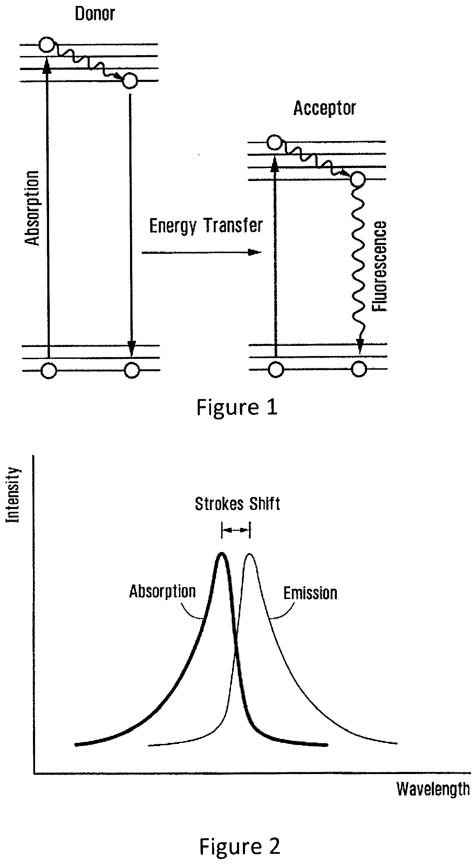

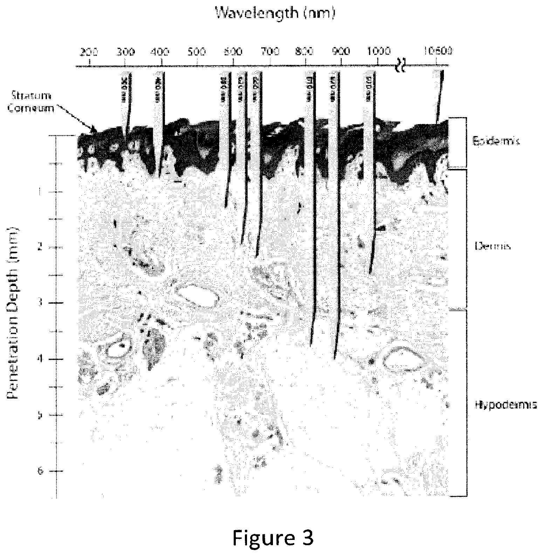

As illustrated in the Jablonski diagram of **Figure 1****,** when a chromophore absorbs a photon of a certain wavelength, it becomes excited. This is an unstable condition and the molecule tries to return to the ground state, giving away the excess energy. For some chromophores, it is favorable to emit the excess energy as light when returning back to the ground state. This process is called fluorescence. The peak wavelength of the emitted fluorescence is shifted towards longer wavelengths compared to the absorption wavelengths due to loss of energy in the conversion process. This is called the Stokes' shift and is illustrated in **Figure 2****.** The emitted light may be transferred to the treatment site where it may have a beneficial effect. Different wavelengths of light have been shown to have different depths of penetration in the skin **(****Figure 3****).** The excess energy may also cause photochemical activation of other agents contained in the composition or at the site of topical application. For example, the energy may be transferred to oxygen molecules to create active oxygen species.

The amount of energy that can be absorbed by a chromophore is proportional to the intensity of the incident light at the wavelength range at which the chromophore absorbs. Sunlight, overhead lighting and white light generally have broad wavelength spectra and the chromophore will absorb the wavelengths of the broad range relevant to its absorption spectra which may not have the highest intensity. Therefore, direct light of a narrower more appropriate wavelength can photoexcite a chromophore more efficiently than white light. However, without being held to theory, it is believed that ambient light, or a combination of ambient light and direct light (including sunlight and white light) can photoactivate chromophores over a longer duration of time to generate low level photoemission and reactive oxygen species or free radical generation. Photobleaching in this case is slower.

It is believed that low levels of reactive oxygen species (especially singlet oxygen) generated within tissues and low levels of light can have a beneficial hormetic effect on the tissue.

The biophotonic compositions of the present disclosure are for topical uses. These compositions may be described based on the components making up the composition. Additionally or alternatively, the compositions of the present disclosure have functional and structural properties and these properties may also be used to define and describe the compositions.

The compositions according to the present disclosure are provided in a dermatologically acceptable carrier. In some embodiments, the carrier is more or less fluid and may have the appearance of a white or colored cream, an ointment, a milk-like liquid, a lotion, a serum, a paste, or a foam. The composition can optionally be sprayed on the skin, and in this case be in the form of an aerosol or a liquid.

The composition according to the present disclosure can in particular be provided in the form of a product for caring for the skin of the face or body, the lips, the eyelashes, the eyebrows or the scalp; of an aftershave gel or lotion; or of a depilatory cream; of a moisturizer or a barrier cream.

The compositions according to the present disclosure can be substantially opaque (e.g. have a transmittance less than about 20%, less than about 15%, less than about 10%, less than about 5%).

For improved stability, the composition can be provided in light-proof dark sealed containers. The containers can be jars, pouches, sprays, or the like.

Individual components of the composition according to various embodiments of the present disclosure are detailed below.

### Chromophores

The compositions of the present disclosure comprise one or more chromophores.

Suitable chromophores can be fluorescent dyes (or stains), although other dye groups or dyes (biological and histological dyes, natural dyes, food colorings, carotenoids) can also be used.

In some embodiments, the first chromophore absorbs at a wavelength in the range of the visible spectrum, such as at a wavelength of about 380-800 nm, 380-700, or 380-600 nm. In other embodiments, the first chromophore absorbs at a wavelength of about 200-800 nm, 200-700 nm, 200-600 nm or 200-500 nm. In one embodiment, the first chromophore absorbs at a wavelength of about 200-600 nm. In some embodiments, the first chromophore absorbs light at a wavelength of about 200-300 nm, 250-350 nm, 300-400 nm, 350-450 nm, 400-500 nm, 450-650 nm, 600-700 nm, 650-750 nm or 700-800 nm.

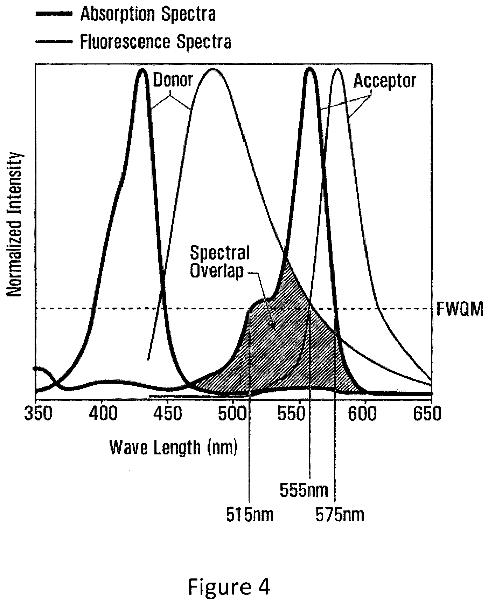

The cosmetic biophotonic compositions disclosed herein may include at least one additional chromophore. When such multichromophore compositions are illuminated with light, energy transfer can occur between the chromophores. This process, known as resonance energy transfer, is a photophysical process through which an excited 'donor' chromophore (also referred to herein as first chromophore) transfers its excitation energy to an 'acceptor' chromophore (also referred to herein as second chromophore). The efficiency and directedness of resonance energy transfer depends on the spectral features of donor and acceptor chromophores. In particular, the flow of energy between chromophores is dependent on a spectral overlap reflecting the relative positioning and shapes of the absorption and emission spectra. For energy transfer to occur, the emission spectrum of the donor chromophore should overlap with the absorption spectrum of the acceptor chromophore. % spectral overlap, as used herein, means the % overlap of a donor chromophore's emission wavelength range with an acceptor chromophore's absorption wavelength range, measured at spectral full width quarter maximum (FWQM). For example, **Figure 4** shows the normalized absorption and emission spectra of donor and acceptor chromophores. The spectral FWQM of the acceptor chromophore's absorption spectrum is from about 60 nm (515 nm to about 575 nm). The overlap of the donor chromophore's spectrum with the absorption spectrum of the acceptor chromophore is about 40 nm (from 515 nm to about 555 nm). Thus, the % overlap can be calculated as 40nm / 60nm x 100 = 66.6%.

Energy transfer manifests itself through decrease or quenching of the donor emission and a reduction of excited state lifetime accompanied also by an increase in acceptor emission intensity.

To enhance the energy transfer efficiency, the donor chromophore should have good abilities to absorb photons and emit photons. Furthermore, it is thought that the more overlap there is between the donor chromophore's emission spectra and the acceptor chromophore's absorption spectra, the better a donor chromophore can transfer energy to the acceptor chromophore.

Combining chromophores may increase photo-absorption by the combined dye molecules and enhance absorption and photo-biomodulation selectivity. This creates multiple possibilities of generating new photosensitive, and/or selective chromophore mixtures.

In certain embodiments where the composition of the present disclosure further comprises a second chromophore, the first chromophore has an emission spectrum that overlaps at least about 80%, 50%, 40%, 30%, 20% or 10% with an absorption spectrum of the second chromophore. In one embodiment, the first chromophore has an emission spectrum that overlaps at least about 20% with an absorption spectrum of the second chromophore. In some embodiments, the first chromophore has an emission spectrum that overlaps at least 1-10%, 5-15%, 10-20%, 15-25%, 20-30%, 25-35%, 30-40%, 35-45%, 50-60%, 55-65% or 60-70% with an absorption spectrum of the second chromophore.

In some embodiments, the second chromophore absorbs at a wavelength in the range of the visible spectrum, or the ultraviolet spectrum. In certain embodiments, the second chromophore has an absorption wavelength that is relatively longer than that of the first chromophore within the range of about 50-250, 25-150 or 10-100 nm.

As discussed above, exposure of the compositions of the present disclosure to light, including ambient light and direct light, results in absorption of light by one or both of the first and second chromophores. In certain embodiments, one of the chromophores absorbs the light and transfers the energy to the other chromophore(s). In certain embodiments, such a cascade of energy transfer provides photons that penetrate the epidermis and/or dermis at the target tissue, including, such as, a site of skin. In some embodiments, such a cascade of energy is transferred to chemical processes resulting in the production of chemical species with therapeutic effect. In other embodiments, such an energy transfer is not accompanied by concomitant generation of heat. In some other embodiments, the cascade of energy transfer does not result in tissue damage.

When the biophotonic topical composition comprises a first and a second chromophore, the first chromophore is present in an amount of about 0.001-40%, preferably about 0.001-20%, more preferably about 0.001-10%, more preferably about 0.001-5%, more preferably about 0.001-1%.per weight of the composition, and the second chromophore is present in an amount of about 0.0003-40%, preferably about 0.0003-20%, more preferably about 0.0003-10%, more preferably about 0.0003-5%, more preferably about 0.0003-1%.per weight of the composition. In certain embodiments, the first chromophore is present in an amount of about 0.001%-0.01%, 0.001-0.1%, 0.0015-1%, 0.5-2%, 1-5%, 2.5-7.5%, 5-10%, 7.5-12.5%, 10-15%, 12.5-17.5%, 15-20%, 17.5-22.5%, 20-25%, 22.5-27.5%, 25-30%, 27.5-32.5%, 30-35%, 32.5-37.5%, or 35-40% per weight of the composition. In certain embodiments, the second chromophore is present in an amount of about 0.0003-0.001%, 0.05-1%, 0.5-2%, 1-5%, 2.5-7.5%, 5-10%, 7.5-12.5%, 10-15%, 12.5-17.5%, 15-20%, 17.5-22.5%, 20-25%, 22.5-27.5%, 25-30%, 27.5-32.5%, 30-35%, 32.5-37.5%, or 35-40% per weight of the composition. In certain embodiments, the total weight per weight of chromophore or combination of chromophores may be in the amount of about 0.0013%-0.01%, 0.05-1%, 0.5-2%, 1-5%, 2.5-7.5%, 5-10%, 7.5-12.5%, 10-15%, 12.5-17.5%, 15-20%, 17.5-22.5%, 20-25%, 22.5-27.5%, 25-30%, 27.5-32.5%, 30-35%, 32.5-37.5%, or 35-40.05% per weight of the composition.

In other embodiments, further chromophore(s) can be provided in the composition which do not have a synergistic effect, and which can absorb light at a wavelength in the range of the ultraviolet spectrum, such as at a wavelength of about 10-400nm. These chromophores can be naturally derived chromophores.

Suitable chromophores that may be used in the compositions of the present disclosure include, but are not limited to the following:

### Chlorophyll dyes

Exemplary chlorophyll dyes include but are not limited to chlorophyll a; chlorophyll b; oil soluble chlorophyll; bacteriochlorophyll a; bacteriochlorophyll b; bacteriochlorophyll c; bacteriochlorophyll d; protochlorophyll; protochlorophyll a; amphiphilic chlorophyll derivative 1; amphiphilic chlorophyll derivative 2, and phycobilins.

### Xanthene derivatives

Exemplary xanthene dyes include but are not limited to Eosin B (4',5'-dibromo,2',7'-dinitr- o-fluorescein, dianion); eosin Y; eosin Y (2',4',5',7'-tetrabromo-fluoresc- ein, dianion); eosin (2',4',5',7'-tetrabromo-fluorescein, dianion); eosin (2',4',5',7'-tetrabromo-fluorescein, dianion) methyl ester; eosin (2',4',5',7'-tetrabromo-fluorescein, monoanion) p-isopropylbenzyl ester; eosin derivative (2',7'-dibromo-fluorescein, dianion); eosin derivative (4',5'-dibromo-fluorescein, dianion); eosin derivative (2',7'-dichloro-fluorescein, dianion); eosin derivative (4',5'-dichloro-fluorescein, dianion); eosin derivative (2',7'-diiodo-fluorescein, dianion); eosin derivative (4',5'-diiodo-fluorescein, dianion); eosin derivative (tribromo-fluorescein, dianion); eosin derivative (2',4',5',7'-tetrachlor- o-fluorescein, dianion); eosin; eosin dicetylpyridinium chloride ion pair; erythrosin B (2',4',5',7'-tetraiodo-fluorescein, dianion); erythrosin; erythrosin dianion; erythiosin B; fluorescein; fluorescein dianion; phloxin B (2',4',5',7'-tetrabromo-3,4,5,6-tetrachloro-fluorescein, dianion); phloxin B (tetrachloro-tetrabromo-fluorescein); phloxine B; rose bengal (3,4,5,6-tetrachloro-2',4',5',7'-tetraiodofluorescein, dianion); pyronin G, pyronin J, pyronin Y; Rhodamine dyes such as rhodamines include 4,5-dibromo-rhodamine methyl ester; 4,5-dibromo-rhodamine n-butyl ester; rhodamine 101 methyl ester; rhodamine 123; rhodamine 6G; rhodamine 6G hexyl ester; tetrabromo-rhodamine 123; and tetramethylrhodamine ethyl ester.

### Methylene blue dyes

Exemplary methylene blue derivatives include but are not limited to 1-methyl methylene blue; 1,9-dimethyl methylene blue; methylene blue; methylene blue (16 µM); methylene blue (14 µM); methylene violet; bromomethylene violet; 4-iodomethylene violet; 1,9-dimethyl-3-dimethyl-amino-7-diethyl-a- mino-phenothiazine; and 1,9-dimetbyl-3-diethylamino-7-dibutyl-amino-phenot- hiazine.

### Azo dyes

Exemplary azo (or diazo-) dyes include but are not limited to methyl violet, neutral red, para red (pigment red 1), amaranth (Azorubine S), Carmoisine (azorubine, food red 3, acid red 14), allura red AC (FD&C 40), tartrazine (FD&C Yellow 5), orange G (acid orange 10), Ponceau 4R (food red 7), methyl red (acid red 2), and murexide-ammonium purpurate.

In some aspects of the disclosure, the one or more chromophores of the biophotonic composition disclosed herein can be independently selected from any of Acid black 1, Acid blue 22, Acid blue 93, Acid fuchsin, Acid green, Acid green 1, Acid green 5, Acid magenta, Acid orange 10, Acid red 26, Acid red 29, Acid red 44, Acid red 51, Acid red 66, Acid red 87, Acid red 91, Acid red 92, Acid red 94, Acid red 101, Acid red 103, Acid roseine, Acid rubin, Acid violet 19, Acid yellow 1, Acid yellow 9, Acid yellow 23, Acid yellow 24, Acid yellow 36, Acid yellow 73, Acid yellow S, Acridine orange, Acriflavine, Alcian blue, Alcian yellow, Alcohol soluble eosin, Alizarin, Alizarin blue 2RC, Alizarin carmine, Alizarin cyanin BBS, Alizarol cyanin R, Alizarin red S, Alizarin purpurin, Allophycocyanin (APC),Aluminon, Amido black 10B, Amidoschwarz, Aniline blue WS, Anthracene blue SWR, Auramine O, Azocannine B, Azocarmine G, Azoic diazo 5, Azoic diazo 48, Azure A, Azure B, Azure C, Basic blue 8, Basic blue 9, Basic blue 12, Basic blue 15, Basic blue 17, Basic blue 20, Basic blue 26, Basic brown 1, Basic fuchsin, Basic green 4, Basic orange 14, Basic red 2 (saffranin O), Basic red 5, Basic red 9, Basic violet 2, Basic violet 3, Basic violet 4, Basic violet 10, Basic violet 14, Basic yellow 1, Basic yellow 2, Biebrich scarlet, Bismarck brown Y, Brilliant crystal scarlet 6R, Calcium red, Carmine, Carminic acid (acid red 4), Celestine blue B, China blue, Cochineal, Coelestine blue, Chrome violet CG, Chromotrope 2R, Chromoxane cyanin R, Congo corinth, Congo red, Cotton blue, Cotton red, Croceine scarlet, Crocin, Crystal ponceau 6R, Crystal violet, Dahlia, Diamond green B, Direct blue 14, Direct blue 58, Direct red, Direct red 10, Direct red 28, Direct red 80, Direct yellow 7, Eosin B, Eosin Bluish, Eosin, Eosin Y, Eosin yellowish, Eosinol, Erie garnet B, Eriochrome cyanin R, Erythrosin B, Ethyl eosin, Ethyl green, Ethyl violet, Evans blue, Fast blue B, Fast green FCF, Fast red B, Fast yellow, Fluorescein, Food green 3, Gallein, Gallamine blue, Gallocyanin, Gentian violet, Haematein, Haematine, Haematoxylin, Helio fast rubin BBL, Helvetia blue, Hematein, Hematine, Hematoxylin, Hoffman's violet, Imperial red, Indocyanin Green, Ingrain blue, Ingrain blue 1, Ingrain yellow 1, INT, Kermes, Kermesic acid, Kernechtrot, Lac, Laccaic acid, Lauth's violet, Light green, Lissamine green SF, Luxol fast blue, Magenta 0, Magenta I, Magenta II, Magenta III, Malachite green, Manchester brown, Martius yellow, Merbromin, Mercurochrome, Metanil yellow, Methylene azure A, Methylene azure B, Methylene azure C, Methylene blue, Methyl blue, Methyl green, Methyl violet, Methyl violet 2B, Methyl violet 10B, Mordant blue 3, Mordant blue 10, Mordant blue 14, Mordant blue 23, Mordant blue 32, Mordant blue 45, Mordant red 3, Mordant red 11, Mordant violet 25, Mordant violet 39 Naphthol blue black, Naphthol green B, Naphthol yellow S, Natural black 1, Natural red, Natural red 3, Natural red 4, Natural red 8, Natural red 16, Natural red 25, Natural red 28, Natural yellow 6, NBT, Neutral red, New fuchsin, Niagara blue 3B, Night blue, Nile blue, Nile blue A, Nile blue oxazone, Nile blue sulphate, Nile red, Nitro BT, Nitro blue tetrazolium, Nuclear fast red, Oil red O, Orange G, Orcein, Pararosanilin, Phloxine B, Picric acid, Ponceau 2R, Ponceau 6R, Ponceau B, Ponceau de Xylidine, Ponceau S, Primula, Purpurin, Phycocyanins, Phycoerythrins. Phycoerythrincyanin (PEC), Phthalocyanines, Pyronin B, Pyronin G, Pyronin Y, Rhodamine B, Rosanilin, Rose bengal, Saffron, Safranin O, Scarlet R, Scarlet red, Scharlach R, Shellac, Sirius red F3B, Solochrome cyanin R, Soluble blue, Solvent black 3, Solvent blue 38, Solvent red 23, Solvent red 24, Solvent red 27, Solvent red 45, Solvent yellow 94, Spirit soluble eosin, Sudan III, Sudan IV, Sudan black B, Sulfur yellow S, Swiss blue, Tartrazine, Thioflavine S, Thioflavine T, Thionin, Toluidine blue, Toluyline red, Tropaeolin G, Trypaflavine, Trypan blue, Uranin, Victoria blue 4R, Victoria blue B, Victoria green B, Water blue I, Water soluble eosin, Xylidine ponceau, or Yellowish eosin.

In certain embodiments, the composition of the present disclosure includes any one or more of the chromophores listed above, or a combination thereof, so as to provide a biophotonic impact at the application site. In other words, chromophores are used in the composition of the present disclosure to promote skin rejuvenation and/or skin conditioning and/or skin condition maintenance. This is a distinct application of these agents and differs from the use of chromophores as simple stains, or as a catalyst for photo-polymerization, or as photodynamic therapy agents which are taken up into live cells or which sensitize live cells.

In some embodiments, the first chromophore is a non-toxic dye which can be well tolerated by the skin. In certain embodiments, the first chromophore is a xanthene dye. In other embodiments, the composition includes chromophores which may not be as well tolerated by the skin. In this case, the chromophores can be coated or encapsulated in a manner that retains their photoactive nature but avoids or limits contact with the skin/tissue.

In some embodiments, the composition includes Eosin Y as a first chromophore and any one or more of Rose Bengal, Erythrosin, Fluorescein, Phloxine B as a second chromophore. It is believed that these combinations have a synergistic effect as Eosin Y can transfer energy to Rose Bengal, Erythrosin or Phloxine B, and Fluorescein can transfer energy to Eosin Y, Phloxine B and Rose Bengal when activated. This transferred energy is then emitted as fluorescence or results in production of reactive oxygen species. This absorbed and re-emitted light is thought to be transmitted throughout the composition, and also to be transmitted into the site of treatment. It will be appreciated that chromophores must be present in appropriate concentrations (proximity) in order for energy transfer to occur.

In further embodiments, the composition includes the following synergistic combinations: Eosin Y and Fluorescein; Eosin Y and Rose Bengal; Fluorescein and Rose Bengal; Erythrosine in combination with Eosin Y, Rose Bengal or Fluorescein; Phloxine B in combination with one or more of Eosin Y, Rose Bengal, Fluorescein and Erythrosine. Other synergistic chromophore combinations are also possible.

It is thought that at least some of these combinations have a synergistic effect at certain concentration ratios within the composition. For example, at certain concentration ratios and with an appropriate activating light, Eosin Y can transfer energy to Rose Bengal, Erythrosin B or Phloxine B when activated. This transferred energy is then emitted as fluorescence and/or by production of reactive oxygen species (such as singlet oxygen).

The synergistic effect may be apparent by the composition having a light absorption spectrum which spans a broader range of wavelengths compared to an individual light absorption spectrum of one of the individual chromophores in the composition, when the individual chromophores and the composition are activated by the same activating light (light having substantially the same emission spectra). This may confer on the composition the ability to be activated by a broader range of activating light wavelengths, for example by white light avoiding the need for a precise wavelength of activating light.

The synergistic effect may also be evident through the composition having a light emission spectrum which spans a broader range of wavelengths compared to an individual light absorption spectrum of one of the individual chromophores in the composition, when the individual chromophores and the composition are activated by the same activating light. This absorbed and re-emitted light spectrum is thought to be transmitted throughout the composition, and also to be transmitted into the site of treatment. This emitted spectrum will then illuminate the target tissue with different penetration depths **(****Figure 3**), which may confer on the target tissue beneficial therapeutic effects. For example green light has been reported to have wound healing properties. By emitting a broader range of wavelengths, a broader range of therapeutic effects can be achieved. The emitted wavelength can be fine-tuned using different chromophore combinations and concentrations.

The synergistic effect may also be evident through the composition having a higher light absorption or emission peak compared to an individual light absorption/emission peak of one of the individual chromophores in the composition, when the individual chromophores and the composition are activated by the same activating light. The ability to absorb and emit higher levels of photons may have a therapeutic effect in certain applications. Furthermore, less concentration of an individual chromophore may be required to achieve a certain power density. Higher power densities can equate to shorter treatment times.

The synergistic effect may also be evident through the composition producing more oxygen species, in the presence of an oxygen-releasing agent, compared to oxygen species produced by an individual chromophores in the composition, when the individual chromophores and the composition are activated by the same activating light. The ability to produce higher levels of oxygen species without the need to extend treatment time or increase the power density of the activating light may be advantageous in certain situations.

By means of synergistic effects of the xanthene dye combinations in the composition, xanthene dyes which cannot normally be activated by an activating light (such as a blue light) can be activated through energy transfer from xanthene dyes which are activated by the activating light. In this way, the different properties of photoactivated xanthene dyes can be harnessed and tailored according to the cosmetic or the medical therapy required.

For example, Rose Bengal can generate a high yield of singlet oxygen when photoactivated in the presence of molecular oxygen, however it has a low quantum yield in terms of emitted fluorescent light. Rose Bengal has a peak absorption around 540 nm and so is normally activated by green light. Eosin Y has a high fluorescence quantum yield and can be activated by blue light. By combining Rose Bengal with Eosin Y, one obtains a composition which can emit therapeutic fluorescent light and generate singlet oxygen when activated by blue light. In this case, the blue light is thought to photoactivate Eosin Y which transfers some of its energy to Rose Bengal as well as emitting some energy as fluorescence.

One or more of the chromophores may photobleach during illumination. This can be a visible confirmation of 'dose' delivery. As the chromophores photobleach, they emit less fluorescence over time. At the same time, they also absorb less of the activating light over time and so the tissues receive increasingly higher amounts of the activating light. In this way, the chromophores modulate exposure of the tissue to the light which may provide a somewhat protective effect.

Other synergistic or non-synergistic chromophore combinations are also possible.

The composition of the present disclosure can also include, in addition to the chromophore(s), other cosmetic, dermatological, and pharmaceutical active agents, including, but are not limited to: pro-collagen agents; agents which stimulate the development of the dermal/lipid layers, agents which improve firmness and elasticity, healing factors; antioxidants; free radical scavengers; moisturizers; depigmentation agents; humectants; antimicrobial (e.g., antibacterial) agents; allergy inhibitors; anti-viral agents, anti-parasitic agents, anti-acne agents; anti-aging agents; anti-wrinkling agents, agents which reduce the appearance of fine lines, wrinkles and/or stretch marks, antiseptics; analgesics; anti-inflammatory agents; healing agents; inflammation inhibitors; anti-inflammatory agents, vasoconstrictors; vasodilators; peptides, polypeptides and proteins; deodorants and antiperspirants; skin emollients and skin moisturizers; skin lightening agents; antifungals; depilating agents; counterirritants; poison ivy products; poison oak products; burn products; make-up preparations; emulsifiers, vitamins; amino acids and their derivatives; herbal extracts; flavoids; sensory markers (i.e., cooling agents, heating agents, etc.); skin conditioners; agents to reduce skin pigmentation (e.g. Tyrostat-9 (Glycerin, Rumex Occidentalis extract), skin rejuvenators, chelating agents; cell turnover enhancers; cellular energy providers, coloring agents; UV protection, sunscreens; fragrance, nourishing agents; moisture absorbers; sebum absorbers and the like; skin penetration enhancers; emollients, chelating agents, moisturizers, slip agents and other active ingredients. These agents should be selected such that they do not interfere detrimentally with the light absorption/emission function of the chromophore(s). In certain embodiments, quenching ingredients may be avoided from the composition.

Additional components that may be included in the composition also include:

### Collagens and Agents that Promote Collagen Synthesis

Collagen is a fibrous protein produced in dermal fibroblast cells and forming 70% of the dermis. Collagen is responsible for the smoothing and firming of the skin. Therefore, when the synthesis of collagen is reduced, skin aging will occur, and so the firming and smoothing of the skin will be rapidly reduced. As a result, the skin will be flaccid and wrinkled. On the other hand, when metabolism of collagen is activated by the stimulation of collagen synthesis in the skin, the components of dermal matrices will be increased, leading to effects, such as wrinkle improvement, firmness improvement and skin strengthening. Thus, collagens and agents that promote collagen synthesis may also be useful in the present disclosure. Agents that promote collagen synthesis (i.e., pro-collagen synthesis agents) include amino acids, peptides, proteins, lipids, small chemical molecules, natural products and extracts from natural products.

For instance, it was discovered that intake of vitamin C, iron, and collagen can effectively increase the amount of collagen in skin or bone. See, e.g., U.S. Patent Application Publication 20090069217. Examples of the vitamin C include an ascorbic acid derivative such as L-ascorbic acid or sodium L-ascorbate, an ascorbic acid preparation obtained by coating ascorbic acid with an emulsifier or the like, and a mixture containing two or more of those vitamin Cs at an arbitrary rate. In addition, natural products containing vitamin C such as acerola and lemon may also be used. Examples of the iron preparation include: an inorganic iron such as ferrous sulfate, sodium ferrous citrate, or ferric pyrophosphate; an organic iron such as heme iron, ferritin iron, or lactoferrin iron; and a mixture containing two or more of those irons at an arbitrary rate. In addition, natural products containing iron such as spinach or liver may also be used. Moreover, examples of the collagen include: an extract obtained by treating bone, skin, or the like of a mammal such as bovine or swine with an acid or alkaline; a peptide obtained by hydrolyzing the extract with a protease such as pepsine, trypsin, or chymotrypsin; and a mixture containing two or more of those collagens at an arbitrary rate. Collagens extracted from plant sources may also be used. Creatine (such as Tego Cosmo C) and Matrixyl 3000 (Glycerin, Butylene Glycol, Carbomer, Polysorbate 20, Palmitoyl Oligopeptide, Palmitoyl Tetrapeptide-7) may also be used to stimulate dermal collagen.

Additional pro-collagen synthesis agents are described, for example, in U.S. Patent Patents 7598291, 7722904, 6203805 , 5529769, etc, and U.S. Patent Application Publications 20060247313,20080108681,20110130459,20090325885,20110086060,etc.

### Healing Factors

Healing factors comprise compounds that promote or enhance the healing or regenerative process of the tissues on the application site of the composition. During the photoactivation of the composition of the present disclosure, for some compositions there is observed an increase of the absorption of molecules at the treatment site by the skin or the mucosa. An augmentation in the blood flow at the site of treatment is also observed for some compositions over an extended period of time. An increase in the lymphatic drainage and a possible change in the osmotic equilibrium due to the dynamic interaction of the free radical cascades can be enhanced or even fortified with the inclusion of healing factors. Suitable healing factors include, but are not limited to:
Hyaluronic acid (Hyaluronan, hyaluronate): is a non-sulfated glycosaminoglycan, distributed widely throughout connective, epithelial and neural tissues. It is one of the primary components of the extracellular matrix, and contributes significantly to cell proliferation and migration. Hyaluronan is a major component of the skin, where it is involved in tissue repair. While it is abundant in extracellular matrices, it contributes to tissues hydrodynamics, movement and proliferation of cells and participates in a wide number of cell surface receptor interactions, notably those including primary receptor CD44. The hyaluronidases enzymes degrade hyaluronan. There are at least seven types of hyaluronidase-like enzymes in humans, several of which are tumor suppressors. The degradation products of hyaluronic acid, the oligosaccharides and the very-low molecular weight hyaluronic acid, exhibit pro-angiogenic properties. In addition, recent studies show that hyaluronan fragments, but not the native high molecular mass of hyaluronan, can induce inflammatory responses in macrophages and dendritic cells in tissue injury. Hyaluronic acid is well suited to biological applications targeting the skin. Due to its high biocompatibility, it is used to stimulate tissue regeneration. Current studies evidenced hyaluronic acid appearing in the early stages of healing to physically create room for white blood cells that mediate the immune response. It is used in the synthesis of biological scaffolds for wound healing applications and in wrinkle treatment.

Glucosamine: is one of the most abundant monosaccharides in human tissues and a precursor in the biological synthesis of glycosilated proteins and lipids. It is commonly used in the treatment of osteoarthritis. The common form of glucosamine used is its sulfate salt. Glucosamine shows a number of effects including an anti-inflammatory activity, stimulation of the synthesis of proteoglycans and the synthesis of proteolytic enzymes. A suitable range of concentration over which glucosamine can be used in the present composition is from about 1% to about 3%. Another form of glucosamine which can be used is acetyl glucosamine.

Allantoin: is a diureide of glyosilic acid. It has keratolytic effect, increases the water content of the extracellular matrix, enhances the desquamation of the upper layers of dead (apoptotic) skin cells, and promotes skin proliferation and wound healing. It is considered as an anti-irritant. Also, saffron can act as both a chromophore and a healing factor.

### Vitamins

Various vitamins can be included in the compositions of the present disclosure. For example, vitamin A and derivatives thereof, vitamin B.sub.2, biotin, pantothenic acid, vitamin K, vitamin D, vitamin E, vitamin C, and mixtures thereof can be used.

### Antimicrobial and Antifungal Actives

Antimicrobials kill microbes or inhibit their growth or accumulation. Exemplary antimicrobials (or antimicrobial agent) are recited in U.S. Patent Application Publications 20040009227 and 20110081530. Suitable antimicrobials for use in the methods of the present disclosure include, but not limited to, oxidants such as carbamide peroxide and hydrogen peroxide, phenolic and chlorinated phenolic and chlorinated phenolic compounds, resorcinol and its derivatives, bisphenolic compounds, benzoic esters (parabens), halogenated carbonilides, Euxyl PE9010 (phenoxyethanol & Ethylhexylglycerin), polymeric antimicrobial agents, thazolines, trichloromethylthioimides, natural antimicrobial agents (also referred to as "natural essential oils"), metal salts, and broad-spectrum antibiotics. An oxidant such as a 0.1% or lower amount of hydrogen peroxide, or its equivalent, can be included in the composition.

Additional non-limiting examples of antimicrobial and antifungal actives include betalactam drugs, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, arnanfadine sulfate, octopirox, parachlorometa xylenol, nystatin, tolnaftate, zinc pyrithione; clotrimazole; alantolactone; isoalantolactone; alkanet extract (alaninin); anise; arnica extract (helenalin acetate and 11, 13 dihydrohelenalin); Aspidium extract (phloro, lucinol containing extract); barberry extract (berberine chloride); bay sweet extract; bayberry bark extract (myricitrin); benzalkonium chloride; benzethonium chloride; benzoic acid and its salts; benzoin; benzyl alcohol; blessed ! 5 thistle; bletilla tuber; bloodroot; bois de rose oil; burdock; butyl paraben; cade oil; CAE (available from Ajinomoto, located in Teaneck, N.J.); cajeput oil; Cangzhu; capsicum frutescens extract; caraway oil; cascarilla bark (sold under the tradename ESSENTIAL OIL); cedarleaf oil; chamomille; chaparral; chlorhexidine gluconate; chlorophenesin; chlorxylenol; cinnamon oil; citronella oil; clove oil; Crinipan AD (available from Climbazole); 2,3-dihydro-farnesol; dehydroacetic acid and its salts; dill seed oil; DOWICIL 200 (available from Dow Chemical, located in Midland, Mich.); echinacea; elenolic acid; epimedium; ethyl paraben; Fo-Ti; galbanum; garden bumet; GERMALL 115 and GERMALL II (available from ISP-Sutton Labs, located in Wayne, N.J.); German chamomile oil; giant knotweed; GLYDANT (available from Lonza, located in Fairlawn, N.J.); GLYDANT PLUS (available from Lonza); grapefruit seed oil; 1,6 hexanediol; hexamidine diisethionate; hinokitiol; honey; honeysuckle flower; hops; immortelle; iodopropynl butyl carbamide (available from Lonza); isobutyl paraben; isopropyl paraben; JM ACTICARE (available from Microbial Systems International, located in Nottingham, NG); juniper berries; KATHON CG (available from Rohm and Haas, located in Philadelphia, Pa.); kojic acid; labdanum; lavender; lemon balm oil; lemon grass; methyl paraben; mint; mume; mustard; myrrh; neem seed oil; ortho phenyl phenol; olive leaf extract (available from Bio Botanica); Argania spinosa (Argan) Oil; Olive oil; parsley; patchouly oil; peony root; 1,2 pentandiol; PHENONIP (available from Nipa Labs, located in Wilmington, Del.); phenoxyethanol; phytosphingosine; pine needle oil; PLANSERVATIVE (available from Campo Research); propyl paraben; purslane; quillaira; rhubarb; rose geranium oil; rosemary; sage; salicylic acid; sassafras; savory; sichuan lovage; sodium meta bisulfite; sodium sulfite; SOPHOLIANCE (available from Soliance, located in Compiegne, France); sorbic acid and its salts; sphingosine; stevia; storax; sucrose esters; tarmic acid; tea; tea tree oil (cajeput oil); thyme; triclosan; triclocarban; tropolone; turpentine; umbelliferone (antifungal); yucca; and mixtures thereof.

Phenolic and chlorinated phenolic antimicrobial agents may also be used. These include but are not limited to: phenol; 2-methyl phenol; 3-methyl phenol; 4-methyl phenol; 4-ethyl phenol; 2,4-dimethyl phenol; 2,5-dimethyl phenol; 3,4-dimethyl phenol; 2,6-dimethyl phenol; 4-n-propyl phenol; 4-n-butyl phenol; 4-n-amyl phenol; 4-tert-amyl phenol; 4-n-hexyl phenol; 4-n-heptyl phenol; mono- and poly-alkyl and aromatic halophenols; p-chlorophenyl; methyl p-chlorophenol; ethyl p-chlorophenol; n-propyl p-chlorophenol; n-butyl p-chlorophenol; n-amyl p-chlorophenol; sec-amyl p-chlorophenol; n-hexyl p-chlorophenol; cyclohexyl p-chlorophenol; n-heptyl p-chlorophenol; n-octyl; p-chlorophenol; o-chlorophenol; methyl o-chlorophenol; ethyl o-chlorophenol; n-propyl o-chlorophenol; n-butyl o-chlorophenol; n-amyl o-chlorophenol; tert-amyl o-chlorophenol; n-hexyl o-chlorophenol; n-heptyl o-chlorophenol; o-benzyl p-chlorophenol; o-benxyl-m-methyl p-chlorophenol; o-benzyl-m,m-dimethyl p-chlorophenol; o-phenylethyl p-chlorophenol; o-phenylethyl-m-methyl p-chlorophenol; 3-methyl p-chlorophenol 3,5-dimethyl p-chlorophenol, 6-ethyl-3-methyl p-chlorophenol, 6-n-propyl-3-methyl p-chlorophenol; 6-iso-propyl-3-methyl p-chlorophenol; 2-ethyl-3,5-dimethyl p-chlorophenol; 6-sec-butyl-3-methyl p-chlorophenol; 2-iso-propyl-3,5-dimethyl p-chlorophenol; 6-diethylmethyl-3-methyl p-chlorophenol; 6-iso-propyl-2-ethyl-3-methyl p-chlorophenol; 2-sec-amyl-3,5-dimethyl p-chlorophenol; 2-diethylmethyl-3,5-dimethyl p-chlorophenol; 6-sec-octyl-3-methyl p-chlorophenol; p-chloro-m-cresol p-bromophenol; methyl p-bromophenol; ethyl p-bromophenol; n-propyl p-bromophenol; n-butyl p-bromophenol; n-amyl p-bromophenol; sec-amyl p-bromophenol; n-hexyl p-bromophenol; cyclohexyl p-bromophenol; o-bromophenol; tert-amyl o-bromophenol; n-hexyl o-bromophenol; n-propyl-m,m-dimethyl o-bromophenol; 2-phenyl phenol; 4-chloro-2-methyl phenol; 4-chloro-3-methyl phenol; 4-chloro-3,5-dimethyl phenol; 2,4-dichloro-3,5-dimethylphenol; 3,4,5,6-tetabromo-2-methylphenol- ; 5-methyl-2-pentylphenol; 4-isopropyl-3-methylphenol; para-chloro-metaxylenol (PCMX); chlorothymol; phenoxyethanol; phenoxyisopropanol; and 5-chloro-2-hydroxydiphenylmethane.

Resorcinol and its derivatives can also be used as antimicrobial agents. Specific resorcinol derivatives include, but are not limited to: methyl resorcinol; ethyl resorcinol; n-propyl resorcinol; n-butyl resorcinol; n-amyl resorcinol; n-hexyl resorcinol; n-heptyl resorcinol; n-octyl resorcinol; n-nonyl resorcinol; phenyl resorcinol; benzyl resorcinol; phenylethyl resorcinol; phenylpropyl resorcinol; p-chlorobenzyl resorcinol; 5-chloro-2,4-dihydroxydiphenyl methane; 4'-chloro-2,4-dihydroxydiphenyl methane; 5-bromo-2,4-dihydroxydiphenyl methane; and 4'-bromo-2,4-dihydroxydiphenyl methane.

Specific bisphenolic antimicrobial agents that can be used in the disclosure include, but are not limited to: 2,2'-methylene bis-(4-chlorophenol); 2,4,4'trichloro-2'-hydroxy-diphenyl ether, which is sold by Ciba Geigy, Florham Park, N.J. under the tradename Triclosan®; 2,2'-methylene bis-(3,4,6-trichlorophenol); 2,2'-methylene bis-(4-chloro-6-bromophenol); bis-(2-hydroxy-3,5-dichlorop- henyl) sulphide; and bis-(2-hydroxy-5-chlorobenzyl)sulphide.

Specific benzoic esters (parabens) that can be used in the disclosure include, but are not limited to: methylparaben; propylparaben; butylparaben; ethylparaben; isopropylparaben; isobutylparaben; benzylparaben; sodium methylparaben; and sodium propylparaben.

Specific halogenated carbanilides that can be used in the disclosure include, but are not limited to: 3,4,4'-trichlorocarbanilides, such as 3-(4-chlorophenyl)-1-(3,4-dichlorphenyl)urea sold under the tradename Triclocarban® by Ciba-Geigy, Florham Park, N.J.; 3-trifluoromethyl-4,4'-dichlorocarbanilide; and 3,3',4-trichlorocarbanilide.

Specific polymeric antimicrobial agents that can be used in the disclosure include, but are not limited to: polyhexamethylene biguanide hydrochloride; and poly(iminoimidocarbonyl iminoimidocarbonyl iminohexamethylene hydrochloride), which is sold under the tradename Vantocil® IB.

Specific thazolines that can be used in the disclosure include, but are not limited to that sold under the tradename Micro-Check®; and 2-n-octyl-4-isothiazolin-3-one, which is sold under the tradename Vinyzene® IT-3000 DIDP.

Specific natural antimicrobial agents that can be used in the disclosure include, but are not limited to, oils of: anise; lemon; orange; rosemary; wintergreen; thyme; lavender; cloves; hops; tea tree; citronella; wheat; barley; lemongrass; cedar leaf; cedarwood; cinnamon; fleagrass; geranium; sandalwood; violet; cranberry; eucalyptus; vervain; peppermint; gum benzoin; basil; fennel; fir; balsam; menthol; ocmea origanuin; hydastis; carradensis; Berberidaceac daceae; Ratanhiae longa; and Curcuma longa. Also included in this class of natural antimicrobial agents are the key chemical components of the plant oils which have been found to provide antimicrobial benefit. These chemicals include, but are not limited to: anethol; catechole; camphene; thymol; eugenol; eucalyptol; ferulic acid; farnesol; hinokitiol; tropolone; limonene; menthol; methyl salicylate; carvacol; terpineol; verbenone; berberine; ratanhiae extract; caryophellene oxide; citronellic acid; curcumin; nerolidol; and geraniol.

Additional antimicrobial agents that can be used in the methods of the disclosure include those disclosed by U.S. Pat. Nos. 3,141,321; 4,402,959; 4,430,381; 4,533,435; 4,625,026; 4,736,467; 4,855,139; 5,069,907; 5,091,102; 5,639,464; 5,853,883; 5,854,147; 5,894,042; and 5,919,554, and U.S. Pat. Appl. Publ. Nos. 20040009227 and 20110081530.

### Anti-Inflammatory Agents

Anti-inflammatories can be included in the biophotonic composition of the present disclosure. Suitable steroidal anti-inflammatories include hydrocortisone; non-steroidal anti-inflammatories such as oxicans, salicylates, acetic acid derivatives, fenamates, propionic acid derivatives, pyrazoles, substituted phenyl compounds, 2-naphthyl containing compounds, and natural anti-inflammatories such as aloe vera. Honey can also be used and is known for its anti-inflammatory and antioxidant properties. Examples of anti-inflammatories are described in U.S. Pat. No. 5,487,884.

### Anti-Wrinkle and Skin Repair Actives

Anti-wrinkle and skin repair actives can be effective in replenishing or rejuvenating the epidermal layer and can be included in the biophotonic composition of the present disclosure. These actives generally provide these desirable skin care benefits by promoting or maintaining the natural process of desquamation. Nonlimiting examples of anti-wrinkle actives include vitamin B3 compounds (such as niacinamide and nicotinic acid), salicylic acid and derivatives thereof (such as 5-octanoyl salicylic acid, heptyloxy 4 salicylic acid, and 4-methoxy salicylic acid); sulfur-containing D and L amino acids and their derivatives and salts, particularly the N-acetyl derivatives, a preferred example of which is N-acetyl-L-cysteine; chronoline (Glycerin, water, Dextran, caprooyl-Tetrapeptide-3), creatine, thiols, e.g. ethane thiol; hydroxy acids, phytic acid, lipoic acid; lysophosphatidic acid; skin peel agents (e.g., phenol and the like); acetyl glucosamine, Actein 27-Deoxyactein Cimicifugoside (available from Cirnigoside); adapalene; ademethionine; adenosine; aletris extract; alkyl glutathione esters; alkoxyalkoxy alkoxyn benzoic and derivatives; aloe derived lectins; amino propane phosphoric acid; 3-aminopropyl dihydrogen phosphate; Amadorine (available from Barnet Products); anise extracts; AOSINE (available from Secma); arginine amino benzoate; ASC III (available from E. Merck, located in Darmstadt, Germany); ascorbic acid; ascorbyl palmitate; asiatic acid; asiaticosides; ARLAMOL GEO (available from ICI, located in Wilmington, Del.); azaleic acid; benzoic acid derivatives; bertholletia extracts; betulinic acid; BIOCHANIN A AND BIOPEPTIDE CL (available from Sederma, located in Brooklyn, N.Y.); BIOPEPTIDE EL (available from Sederma); biotin; blackberry bark extract; blackberry lily extracts; black cohosh extract; blue cohesh extract; butanoyl betulinic acid; carboxymethyl 1,3 beta glucan; catecholamines; chalcones; citric acid esters; chaste tree extract; clover extracts; coumestrol; CPC Peptide (available from Barnet Products); daidzein; dang gui extract; darutoside; debromo laurinterol; 1-decanoyl-glycero-phosphon- ic acid; dehydrocholesterol; dehydrodicreosol; dehydrodieugenol; dehydroepiandersterone; DERMOLECTINE (available from Sederma); dehydroascorbic acid; dehydroepiandersterone sulfate; dianethole; dihydroxy benzoic acid; 2,4 dihydroxybenzoic acid; diglycol guanidine succinate; diosgenin; disodium ascorbyl phosphate; dodecanedioic acid; Ederline (available from Seporga); Enderline (available from Laboratories Seporga); equol; eriodictyol; estrogen and its derivatives; ETF (available from Laboratories Seporga); ethocyn; ELESERYL SH (available from Laboratories Serobiologiques, located in Somerville, N.J.); ENDONUCLEINE (available from Laboratories Serobiologiques); ergosterol; eythrobic acid; fennel extract; fenugreek seed extract; FIBRASTIL (available from Sederma); FIBROSTIMULINES S and P (available from Sederma); FIRMOGEN LS 8445 (available from Laboratories Serobiologiques); formononetin; forsythia fruit extract; gallic acid esters; gamma amino butyric acid; GATULINE RC (available from Gattlefosse, located in Priest, France); genistein; genisteine; genistic acid; gentisyl alcohol; gingko bilboa extracts; ginseng extracts; ginsenoside; gluco pyranosyl-L-ascorbate; glutathione and its esters; glycitein; hesperitin; hexahydro curcumin; HMG-coenzyme A reductase inhibitors; hops extracts; 11 hydroxy undecanoic acid; 10 hydroxy decanoic acid; 25-hydroxycholesterol; 7-hydroxylated sterols; hydroxyethyl isostearyloxy isopropanolamine; hydroxy-tetra methyl piperidinyloxy; hypotaurine; ibukijakou extract; isoflavone SG 10 (available from Barnet Products); kinetin; kohki extract; L-2-OXO-thiazolidine-4-carboxylic acid esters; lactate dehydrogenase inhibitors; 1-lauryl, -lyso-phosphatidyl choline; lectins; lichochalcone LF15 (available from Maruzen); licorice extracts; lignan; lumisterol; lupenes; luteolin; lysophosphitidic acid; magnesium ascorbyl phosphate; margin; melatonin; melibiose; metalloproteinase inhibitors; methoprene; methoprenic acid; mevalonic acid; MPC COMPLEX (available from CLR); N methyl serine; N methyl taurine; N,N¹bis (lactyl) cysteamine; naringenin; neotigogenin; o-desmethylangoiensin; oat beta glucan; oleanolic acid; pantethine; phenylalanine; photoanethone; piperdine; placental extracts; pratensein; pregnenolone; pregnenolone acetate; pregnenolone succinate; premarin; quillaic acid; raloxifene; REPAIR FACTOR 1 and REPAIR FACTOR FCP (both available from Sederma); retinoates; retinyl glucuronate; retinyl linoleate; S-carboxymethyl cysteine; SEANAMINE FP (available from Laboratories Serobiologiques); sodium ascorbyl phosphate; Songa (Solidago Virgaurea extract), soya extracts; spleen extracts; tachysterol; taurine; tazarotene; tempol; thymulen; thymus extracts; thyroid hormones; tigogenin; tocopheryl retinoate; toxifolin; traumatic acid; tricholine citrate; trifoside; Drieline IS (Sorbitol, Yeast extract), Ubiquinone, uracil derivatives; ursolic acid; vitamin D3 and its analogs; vitamin K; vitex extract; yam extract; yamogenin; zeatin; and mixtures thereof.

### Skin Barrier Repair Actives

Skin barrier repair actives are those skin care actives which can help repair and replenish the natural moisture barrier function of the epidermis and can be included in the biophotonic composition. Non-limiting examples of skin barrier repair actives include Alpha Lipid (available from Lucas Meyer); ascorbic acid; biotin; biotin esters; brassicasterol; caffeine; campesterol; canola derived sterols; Cennamides (available from Ennagram); Ceramax (available from Alban Muller); CERAMAX (available from Quest, located in Ashford, England); CERAMIDE 2 and CERAMIDE HO3 (both available from Sederma); CERAMIDE II (available from Quest); CERAMIDE III and IIIB (both available from Cosmoferm, located in Deft, Netherlands); CERAMIDE LS 3773 (available from Laboratories Serobiologiques); CERAMINOL (available from Inocosm); Cerasol and Cephalip (both available from Pentapharm); cholesterol; cholesterol hydroxystearate; cholesterol isostearate; 7 dehydrocholesterol; DERMATEIN BRC and DERMATEIN GSL (both available from Hormel); ELDEW CL 301 AND ELDEW PS 203 (both available from Ajinomoto); Fitobroside (available from Pentapharm); galactocerebrosides; Generol 122 (available from Henkel); glyceryl serine amide; hydroxyethyl isostearyl isopropanolamine; lactic acid; Lactomide (available from Pentapharm); lanolin; lanolin alcohols; lanosterol; lauric acid N laurylglucamide; lipoic acid; N-acetyl cysteine; N-acetyl-L-serine; N-methyl-L-Serine; Net Sterol-ISO (available from Barnet Products); vitamin B3 compounds (such as niacinamide and nicotinic acid); palmitic acid; panthenol; panthetine; phosphodiesterase inhibitors; PHYTO/CER (available from Intergen); phytoglycolipid millet extract (available from Barnet Products Distributer, located in Englewood, N.J.); PHYTOSPHINGOSINE (available from Gist Brocades, located in King of Prussia, Pa.); PSENDOFILAGGRIN (available from Brooks Industries, located in South Plainfield, N.J.); QUESTAMIDE H (available from Quest); serine; sigmasterol; sitosterol; soybean derived sterols; sphingosine; sphingomylinase; S-lactoyl glutathione; stearic acid; Structurine (available from Silah); SUPER STEROL ESTERS (available from Croda); thioctic acid; THSC CERAMIDE OIL (available from Campo Research); trimethyl glycine; tocopheryl nicotinate; vitamin D3; Y2 (available from Ocean Pharmaceutical); and mixtures thereof.

### Non-steroidal Cosmetic Soothing Actives

Cosmetic soothing actives can be effective in preventing or treating inflammation of the skin and can be included in composition of the present disclosure. The soothing active enhances the skin appearance benefits of the provided by the present composition, e.g., such agents contribute to a more uniform and acceptable skin tone or color. The exact amount of anti-inflammatory agent to be used in the compositions will depend on the particular anti-inflammatory agent utilized since such agents vary widely in potency. Non-limiting examples of cosmetic soothing agents include the following categories: propionic acid derivatives; acetic acid derivatives; fenamic acid derivatives; biphenylcarboxylic acid derivatives; and oxicams. Non-limiting examples of useful cosmetic soothing actives include acetyl salicylic acid, ibuprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen, bucloxic acid, absinthium, acacia, aescin, alder buckthorn extract, allantoin, aloe, APT (available from Centerchem), arnica, astragalus, astragalus root extract, azulene, Baicalin SR 15 (available from Bamet Products Dist.), baikal skullcap, baizhu, balsam canada, bee pollen, BIOPHYTEX (available from Laboratories Serobiologiques), bisabolol, black cohosh, black cohosh extract blue cohosh, blue cohosh extract, boneset, borage, borage oil, bradykinin antagonists, bromelain, calendula, calendula extract, Canadian Willowbark Extract (available from Fytokem), candelilla wax, Cangzhu, canola phytosterols, capsicum, carboxypeptidase, celery seed, celery stem extract, CENTAURIUM (available from Sederma), centaury extract, chamazulene, chamomile, chamomile extract, chaparral, chaste tree, chaste tree extract, chickweed, chicory root, chicory root extract, chirata, chishao, collodial oatmeal, comfrey, comfrey extract, CROMOIST CM GLUCAN (available from Croda), darutoside, dehurian angelica, devil's claw, divalent metals (such as, magnesium, strontium, and manganese), doggrass, dogwood, Eashave (available from Pentapharm), eleuthero, ELHIBIN (available from Pentapharm), ENTELINE 2 (available from Secma), ephedra, epimedium, esculoside; etbacrynic acid, evening primrose, eyebright, Extract LE-100 (available from Sino Lion), Fangfeng, feverfew, ficin, forsythia fruit, Fytosterol 85 (available from Fytokem), ganoderma, gaoben, Gatuline A (available from Gattefosse), Emulium (available from Gattefosse), gentian, germanium extract, gingko bilboa extract, ginkgo, ginseng extract, goldenseal, gorgonian extract, gotu kola, grape fruit extract, guaiac wood oil, guggal extract, helenalin esters, henna, honeysuckle flower, horehound extract, horsechestnut, horsetail, huzhang, hypericum, ichthyol, immortelle, LANACHRYS 28 (available from Lana Tech), lemon oil, lianqiao, licorice root, ligusticum, ligustrum, lovage root, luffa, mace, magnolia flower, manjistha extract, margaspidin, matricin, melatonin, MICROAT IRC (available from Nurture), mints, mistletoe, Modulene (available from Seporga), mono or diglucosides of glabridin, mono or diglucosides of gentisin, MTA (5'-deoxy-5'-methythioadenosine), mung bean extract, musk, N-methyl arginine, oat beta glucan, oat extract, orange, panthenol, papain, phenoxyacetic acid, peony bark, peony root, Phytoplenolin (available from Bio Botanica), phytosphingosine, Preregen (available from Pentapharm), purslane, QUENCH T (available from Centerchem), quillaia, red sage, rehmannia, rhubarb, rosemary, rosmarinic acid, royal jelly, rue, rutin, sandlewood, sanqi, sarsaparilla, saw palmetto, SENSILINE (available from Silab), SIEGESBECKIA (available from Sederma), stearyl glycyrrhetinate, Stimutex (available from Pentapharm), storax, strontium nitrate, sweet birch oil, sweet woodruff, tagetes, tea extract, thyme extract, tienchi ginseng, tocopherol, tocopheryl acetate, triclosan, turmeric, urimei, ursolic acid, white pine bark, witch hazel xinyi, yarrow, yeast extract, yucca, and mixtures thereof.

### Sunscreen Actives

Sun screen agents can be included in the composition of the present disclosure. The term "sunscreen agent" as used herein defines ultraviolet ray-blocking compounds exhibiting absorption within the wavelength region between about 290 and about 400 nm. Sunscreens can be classified into five groups based upon their chemical structure: para-amino benzoates; salicylates; cinnamates; benzophenones; and miscellaneous chemicals including menthyl anthranilate and digalloyl trioleate. Inorganic sunscreens can also be used including titanium dioxide, zinc oxide, iron oxide and polymer particles such as those of polyethylene, polymethylmethacrylates and polyamides A wide variety of conventional sunscreening agents are suitable for use in the present composition as described in Segarin et al., at Chapter VIII, Pages 189 et seq., "Cosmetics Science and Technology". Specific suitable sunscreening agents include, for example: p-aminobenzoic acid, its salts and derivatives, anthranilates, salicylates, cinnamic acid derivatives, dihydroxycinnamic acid derivatives, trihydroxycinnamic acid derivatives, hydrocarbons, dibenzalacetone and benzalacetophenone, naphthosulfonates, dihydroxy-naphthoic acid and its salts, o- and p-hydroxy-biphenyldisulfon- ates, coumarin derivatives, diazoles quinine salts, quinoline derivatives, hydroxy or methoxy substituted benzophenones, uric and vilouric acids, tannic acid and its derivatives, hydroquinone, benzophenones, and the like.

Also useful herein are sunscreening actives. A wide variety of sunscreening agents are described in U.S. Pat. No. 5,087,445, to Haffey et al., issued Feb. 11, 1992; U.S. Patent No. 5,073,372, to Turner et al., issued Dec.17, 1991; U.S. Pat. No. 5,073,371, to Turner et al. issued Dec. 17, 1991; and Segarin, et al., at Chapter VIII, pages 189 et seq., of Cosmetics Science and Technology. Non-limiting examples of sunscreens which are useful in the compositions of the present composition are those selected from the group consisting of 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof. Still other useful sunscreens are those disclosed in U.S. Pat. No. 4,937,370, to Sabatelli, issued Jun. 26, 1990; and U.S. Pat. No. 4,999,186, to Sabatelli et al., issued Mar. 12, 1991.

Still other useful sunscreens include para-aminobenzoic acid (PABA), benzylidene camphor, butyl methoxy dibenzoyl methane, diethanolamine p-methoxycinnamate, dioxybenzone, ethyl dihydroxypropyl (PABA), glyceryl aminobenzoate, homomenthyl salicylate, isopropyl dibenzoyl methane, lawsone and dihydroxyacetone, menthyl anthranilate, methyl anthranilate, methyl benzylidene camphor, octocrylene, octyl dimethyl (PABA), octyl methoxycinnamate, oxybenzone, 2-phenylbenzimidazole-5-sulfonic acid, red petrolatum, sulisobenzone, titanium dioxide, triethanolamine salicylate, zinc oxide, Abyssine 657 (Butylene glycol and Alteromonas ferment extract) and mixtures thereof. Examples of these sunscreens include those selected from the group consisting of 4-N,N-(2-ethylhexyl)methylaminobenz- oic acid ester of 2,4-dihydroxybenzophenone, 4-N,N-(2-ethylhexyl)methylami- nobenzoic acid ester with 4-hydroxydibenzoylmethane, 4-N,N-(2-ethylhexyl)-methylaminobenzoic acid ester of 2-hydroxy-4-(2-hydroxyethoxy)benzophenone, 4-N,N-(2-ethylhexyl)-methylaminobenzoic acid ester of 4-(2-hydroxyethoxy)dibenzoylmethane, and mixtures thereof.

Exact amounts of sunscreens which can be employed will vary depending upon the sunscreen chosen and the desired Sun Protection Factor (SPF) to be achieved. SPF is a commonly used measure of photoprotection of a sunscreen against erythema. See Federal Register, Vol. 43, No. 166, pp. 38206-38269, Aug. 25, 1978.

### Skin Conditioners

The composition of the present disclosure can also contain other skin conditioners, moisturizers and surfactants as additives. Illustrative conditioners include mineral oil, petrolatum, vegetable oils (such as soybean or maleated soybean oil), dimethicone, dimethicone copolyol, cationic monomers and polymers (such as guar hydroxypropyl trimonium chloride and distearyl dimethyl ammonium chloride) as well as combinations thereof. Illustrative moisturizers are Propane-1,3-diol, polyols such as sorbitol, glycerin, propylene glycol, ethylene glycol, polyethylene glycol, polypropylene glycol, 1,3-butane diol, hexylene glycol, isoprene glycol, xylitol, fructose, Aqualance (Erythritol, Homarine, HC1), antarcticine with salicyclic acid (Pseudoalteromonas ferment Extract, Salicyclic acid, sodium hydroxide), Cristalhyal FL (Sodium Hyaluronate, 1,2-hexanediol 9, caprylyl glycol), Iricalmin (Triticum Vulgare (wheat) germ extract, saccharomyces Cerevisiae, Sodium Hyaluronate, Panthenol), Nibi (Sambucus Nigra Flower extract), and mixtures thereof.

### Preservatives

Preservatives can desirably be incorporated into the composition of the present disclosure. Suitable preservatives for compositions of the present disclosure include but are not limited to alkyl esters of parahydroxybenzoic acid. Other preservatives, which can be used include hydantoin derivatives, propionate salts, and a variety of quaternary ammonium compounds.

Appropriate preservatives can be selected to satisfy the preservative challenge test and to provide product stability. Particularly preferred preservatives are methylparaben, imidazolidinyl urea, sodium dehydroacetate, propylparaben, trisodium ethylenediamine tetraacetate (EDTA), benzyl alcohol, phenoxyethanol and ethylhexyglycerin. The preservative can be selected based on the consideration of possible incompatibilities between the preservative and other ingredients in the release system.

The compositions of the disclosure may also include gelling agents. Suitable gelling agents for aqueous gels include, but are not limited to, natural gums, polysaccharides, acrylic acid and acrylate polymers and copolymers, and cellulose derivatives (e.g. hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Starches may also be used as the gelling agent.

Certain suitable compositions of the disclosure can be described based on the absence of certain components from the composition. The examples provided herein may be combined so that a suitable composition may specifically exclude one or more of these ingredients. For example, in certain embodiments, the composition does not include polyphenols which can filter UV light. In certain embodiments, the composition does not include self-tanning agents. In certain embodiments, the concentration of the chromophore in the composition is not so high so as to give the composition an unnatural or non-neutral colouring. In certain embodiments, the concentration of the chromophore is not so high so that activation of the chromophore by ambient light or direct light causes the chromophore to visibly fluoresce more than a natural appearing glow.

### (4) Methods of Use

The present disclosure also includes methods of treating skin by topically applying the cosmetic compositions of the present disclosure. In use, a small quantity of the composition, for example from 1 to 100 ml, may be applied to exposed areas of the skin, from a suitable container or applicator and, if necessary, it is then spread over and/or rubbed into the skin using the hand or fingers or a suitable device. The amount of the composition which is applied, the frequency of application and the period of use will vary widely depending upon the active levels of a given composition and the level of regulation desired, e.g., in light of the level of skin ageing present in the subject and the rate of further skin aging.

Some embodiments of the compositions of the present disclosure may provide a visible improvement in skin condition essentially immediately following application of the composition to the skin. Such immediate improvement involves coverage or masking of skin imperfections such as textural discontinuities (including those associated with skin ageing, such as enlarged pores), and/or providing a more even skin tone or color.

The compositions of the present disclosure may also provide visible improvements in skin condition following chronic topical application of the composition. "Chronic topical application" and the like involves continued topical application of the composition over an extended period during the subject's lifetime, for a period of at least about one week, or for a period of at least about one month, or for at least about three months, or for at least about six months, or for at least about one year. Chronic regulation of skin condition involves improvement of skin condition following multiple topical applications of the composition to the skin. Typically applications would be on the order of about once per day over such extended periods, however application rates can vary from about once per week up to about three times per day or more.

Regulating skin condition may be practiced by applying a composition in the form of a skin lotion, cream, cosmetic, or the like which is intended to be left on the skin for an extended period for some aesthetic, prophylactic, therapeutic or other benefit (i.e., a "leave-on" composition). After applying the composition to the skin, the leave-on composition may be left on the skin for a period of at least about 15 minutes, or at least about 30 minutes, or at least about 1 hour, or for at least several hours, e.g., up to about 18 hours or during the waking hours of the user.

A pre-conditioning or post-conditioning composition may be applied to the target tissue, before, during or after the application of the presently disclosed composition. The conditioning composition may be a serum including for example an antioxidant such as Vitamin C, which can be reactive in the presence of some chromophores and therefore advantageous to apply it to the target site separately.

In the methods of the present disclosure, any source of light can be used. For example, a combination of ambient light and direct sunlight or direct artificial light may be used. Ambient light can include overhead lighting such as LED bulbs, halogen lights, fluorescent bulbs etc, and indirect sunlight.

Any type of direct light is suitable for photoactivating the present compositions, for example sunlight or direct light from artificial light sources. For more efficient chromophore activation a halogen, LED or plasma arc lamp, or laser can be used having an emission spectra close to the absorption spectra of the chromophore(s) in the composition. In one embodiment, a laser can be used. In yet another embodiment, a LED lamp is the source of the actinic light. The lamps may be full face or part-face lamps.

In certain embodiments, light emitted from a monitor or telephone/computer screen can be used over a time period to photoactivate the composition. In yet another embodiment, the source of the actinic light is a source of light having a wavelength between about 200 to 600 nm. In another embodiment, the source of the actinic light is a source of visible light having a wavelength between about 400 and 800 nm. Furthermore, the source of actinic light should have a suitable power density. Suitable power density for non-collimated light sources (LED, halogen or plasma lamps) are less than about 200 mW/cm².

In some embodiments of the methods of the present disclosure, the direct light has an energy at the subject's skin surface of between about 0.1 mW/cm² and about 500 mW/cm², or 300 mW/cm², or 200 mW/cm², wherein the energy applied depends at least on the condition being treated and the wavelength of the light. In certain embodiments, the power density of the light irradiating the subject's skin is between about 0.05-1 mW/cm², 1-40 mW/cm², or 20-60 mW/cm², or 40-80 mW/cm², or 60-100 mW/cm², or 80-120 mW/cm², or 100-140 mW/cm², or 120-160 mW/cm², or 140-180 mW/cm², or 160-200 mW/cm², or 110-240 mW/cm², or 110-150 mW/cm², or 190-240 mW/cm².

The duration of the exposure to the direct light source will be dependent on the surface of the treated area, the sensitivity of the skin, the proximity of the skin to the light, the thickness of the composition, the emission spectra of the light. The illumination of the composition may take place within seconds or even fragment of seconds, but a prolonged exposure period is beneficial to exploit the synergistic effects of the absorbed, reflected and reemitted light on the composition of the present disclosure and its interaction with the tissue being treated. In one embodiment, the time of exposure to actinic light of the tissue or skin is a period between 1 minute and 5 minutes. In another embodiment, the time of exposure to actinic light of the tissue or skin is a period between 1 minute and 5 minutes. In some other embodiments, the biophotonic composition is illuminated for a period between 1 minute and 3 minutes. In certain embodiments, light is applied for a period of 1-30 seconds, 15-45 seconds, 30-60 seconds, 0.75-1.5 minutes, 1-2 minutes, 1.5-2.5 minutes, 2-3 minutes, 2.5-3.5 minutes, 3-4 minutes, 3.5-4.5 minutes, 4-5 minutes, 5-10 minutes, 10-15 minutes, 15-20 minutes, or 20-30 minutes. In yet another embodiment, the source of actinic light is in continuous motion over the treated area for the appropriate time of exposure. In yet another embodiment, multiple applications of the skin rejuvenation composition as described herein and actinic light are performed. In some embodiments, the tissue or skin is exposed to actinic light at least two, three, four, five or six times. In some embodiments, a fresh application of the skin rejuvenation composition of the present disclosure is applied before exposure to actinic light.

In the methods of the present disclosure, the biophotonic composition may be optionally removed from the skin following application of light.

In some embodiments, the light which illuminates the biophotonic composition is ambient light including indirect sunlight and overhead light bulbs. In some embodiments, the light which illuminates the biophotonic composition is a combination of ambient light and direct light including sunlight. For example, a user may apply the biophotonic composition to exposed parts of his or her body such as the face, ears, neck, hands, arms, and/or legs and then go about his or her normal activities which may be indoors, outdoors, or a combination of indoor and outdoor activities. In this way, the biophotonic composition on the user's face will be exposed to light from the sun, and/or from other light sources such as overhead lamps.

The method of the present disclosure, in some embodiments, comprises a step of illuminating the biophotonic composition with ambient light, or a combination of ambient and direct light, over a period of at least about 24 hours, at least about 18 hours, at least about 16 hours, at least about 12 hours, at least about 11 hours, at least about 10 hours, at least about 9 hours, at least about 8 hours, at least about 7 hours, at least about 6 hours, at least about 5 hours, at least about 4 hours, at least about 3 hours, at least about 2 hours, at least about 1 hour. In certain other embodiments, the method of the present disclosure comprises a step of illuminating the biophotonic composition with ambient, and/or a combination of ambient and direct light, over a waking period of time of the user of the biophotonic composition. In certain embodiments, the biophotonic composition is removed from the target skin tissue after about 24 hours, 18 hours, 12 hours, 11 hours, 10 hours, 9 hours, 8 hours, 7 hours, 6 hours, 5 hours, 4 hours, 3 hours, 2 hours, or 1 hour. In other embodiments, the biophotonic composition is not removed from the target skin tissue.

### (5) Skin Aging and Rejuvenation

The dermis is the second layer of skin, containing the structural elements of the skin, the connective tissue. There are various types of connective tissue with different functions. Elastin fibers give the skin its elasticity, and collagen gives the skin its strength.

The junction between the dermis and the epidermis is an important structure. The dermal-epidermal junction interlocks forming finger-like epidermal ridges. The cells of the epidermis receive their nutrients from the blood vessels in the dermis. The epidermal ridges increase the surface area of the epidermis that is exposed to these blood vessels and the needed nutrients.

The aging of skin comes with significant physiological changes to the skin. The generation of new skin cells slows down, and the epidermal ridges of the dermal-epidermal junction flatten out. While the number of elastin fibers increases, their structure and coherence decrease. Also the amount of collagen and the thickness of the dermis decrease with the ageing of the skin.

Collagen is a major component of the skin's extracellular matrix, providing a structural framework. During the aging process, the decrease of collagen synthesis and insolubilization of collagen fibers contribute to a thinning of the dermis and loss of the skin's biomechanical properties.

The physiological changes to the skin result in noticeable aging symptoms often referred to as chronological-, intrinsic- and photo-ageing. The skin becomes drier, roughness and scaling increase, the appearance becomes duller, and most obviously fine lines and wrinkles appear. Other symptoms or signs of skin aging include, but are not limited to, thinning and transparent skin, loss of underlying fat (leading to hollowed cheeks and eye sockets as well as noticeable loss of firmness on the hands and neck), bone loss (such that bones shrink away from the skin due to bone loss, which causes sagging skin), dry skin (which might itch), inability to sweat sufficiently to cool the skin, unwanted facial hair, freckles, age spots, spider veins, rough and leathery skin, fine wrinkles that disappear when stretched, loose skin, a blotchy complexion.

The dermal-epidermal junction is a basement membrane that separates the keratinocytes in the epidermis from the extracellular matrix, which lies below in the dermis. This membrane consists of two layers: the basal lamina in contact with the keratinocytes, and the underlying reticular lamina in contact with the extracellular matrix. The basal lamina is rich in collagen type IV and laminin, molecules that play a role in providing a structural network and bioadhesive properties for cell attachment.

Laminin is a glycoprotein that only exists in basement membranes. It is composed of three polypeptide chains (alpha, beta and gamma) arranged in the shape of an asymmetric cross and held together by disulfide bonds. The three chains exist as different subtypes which result in twelve different isoforms for laminin, including Laminin-1 and Laminin-5.

The dermis is anchored to hemidesmosomes, specific junction points located on the keratinocytes, which consist of α-integrins and other proteins, at the basal membrane keratinocytes by type VII collagen fibrils. Laminins, and particularly Laminin-5, constitute the real anchor point between hemidesmosomal transmembrane proteins in basal keratinocytes and type VII collagen.

Laminin-5 synthesis and type VII collagen expression have been proven to decrease in aged skin. This causes a loss of contact between dermis and epidermis, and results in the skin losing elasticity and becoming saggy.

Recently another type of wrinkles, generally referred to as expression wrinkles, got general recognition. These wrinkles require loss of resilience, particularly in the dermis, because of which the skin is no longer able to resume its original state when facial muscles which produce facial expressions exert stress on the skin, resulting in expression wrinkles.

The present disclosure provides compositions and methods for preventing, arresting, reversing, ameliorating, diminishing, reducing or improving a sign of aging, e.g., skin rejuvenation, in which a composition of the present disclosure is topically applied to skin in a cosmetically effective amount sufficient to prevent, arrest, reverse ameliorate, diminish, reduce or improve a sign of aging in skin. Exemplary signs of aging include, but are not limited to, facial lines, fine lines, wrinkles, crow's feet, dark eye circles, blemishes, age spots, stretch marks, or combinations thereof.

The present disclosure also provides compositions and methods for improving the aesthetic appearance of skin, in which a composition of the present disclosure is topically applied to skin in a cosmetically effective amount sufficient to improve the aesthetic appearance of the skin. The improvements may relate to skin thickness, elasticity, resiliency, moisturization, tone, texture, radiance, luster, brightness, clarity, contour, firmness, tautness, suppleness, softness, sensitivity, pore size, or combinations thereof.

In certain embodiments, the compositions and methods of the present disclosure promote collagen synthesis. In certain other embodiments, the compositions and methods of the present disclosure may reduce, diminish, retard or even reverse one or more signs of skin aging including, but not limited to, appearance of fine lines or wrinkles, thin and transparent skin, loss of underlying fat (leading to hollowed cheeks and eye sockets as well as noticeable loss of firmness on the hands and neck), bone loss (such that bones shrink away from the skin due to bone loss, which causes sagging skin), dry skin (which might itch), inability to sweat sufficiently to cool the skin, unwanted facial hair, freckles, age spots, spider veins, rough and leathery skin, fine wrinkles that disappear when stretched, loose skin, or a blotchy complexion. In certain embodiments, the compositions and methods of the present disclosure may induce a reduction in pore size, enhance sculpturing of skin subsections, and/or enhance skin translucence.

Any one or more of the features of the previously described embodiments may be combined in any manner. Many variations of the invention will become apparent to those skilled in the art upon review of the specification. The following example is offered by way of illustration and not by way of limitation.

### EXAMPLES

### Example 1- Absorption/emission spectra of biophotonic cream 'O' before and after illumination

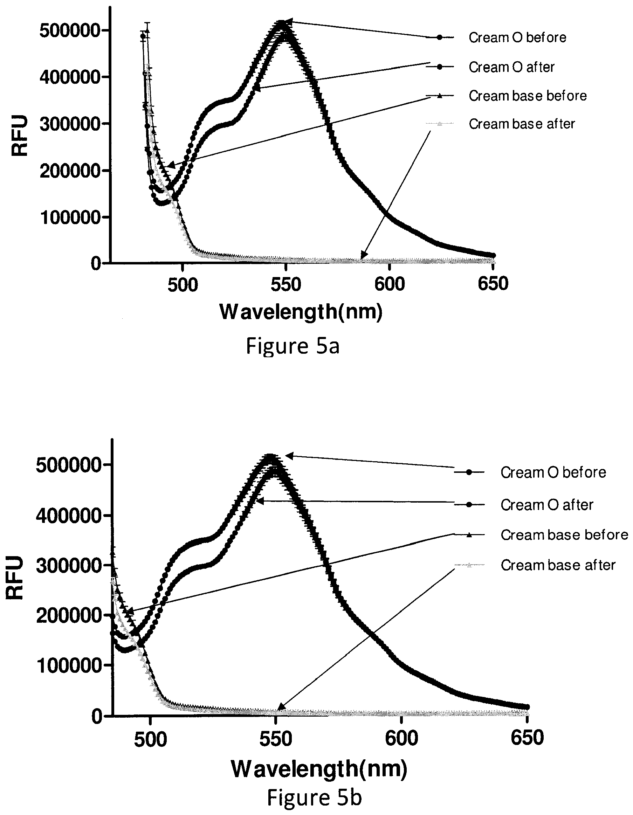

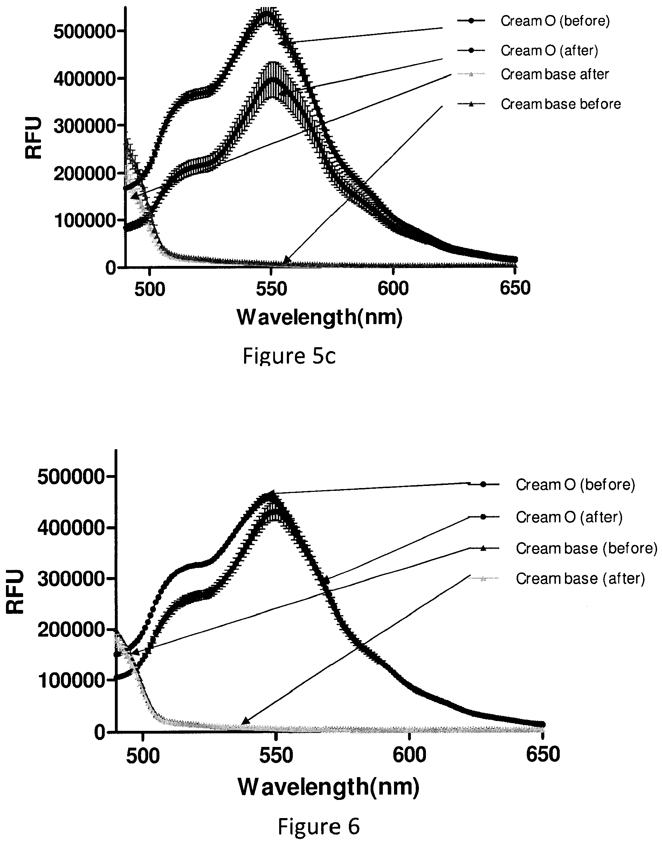

A composition ('Cream 0'), according to an embodiment of the present disclosure, was evaluated for biophotonic activity. Cream 0 comprised a base cream having 0.001% chromophore content. Two mm of Cream 0 was applied to a plastic plate and left on a laboratory bench under ceiling lights (120 cycle overhead lights) for about 3, 6 and 16.5 hours. Cream 0 was placed approximately 182 cm from the ceiling light, and based on the power density of light at this distance, the light was deemed to be ambient light. **Figures 5A, 5B** and **5C** show the emission spectra of the Cream 0 composition and a control cream (base cream alone) left under the ceiling light for 3, 6 and 16.5 hours, compared to the Cream 0 composition and the base cream control at time zero. A flexstation 384 II spectrophotometer was used to measure emitted fluorescence with the following parameters: mode fluorescence, excitation 460 nm, emission spectra 465-750 nm. The fluorescence emitted by the Cream 0 composition after about 3, 6 and 16.5 hours of light exposure was lower than that seen at time zero. This reduction in fluorescence indicates a reduction in biophotonic activity which suggests that the composition was at least partially photobleached through photoactivation by the ceiling light. At least partial photobleaching was observed as a change in colour. The rate of photobleaching (and hence emitted fluorescence) observed with ambient light was lower than that seen when the cream was activated by a light emitting a wavelength of light with a larger overlap with the absorption spectra of the chromophores (e.g. blue light).

The same results were seen when the composition was left under a direct light (a desk lamp having an incandescent light bulb, 40W, 130V) at a distance of about 30 cm for about 3, 6 and 16.5 hours. As an illustrative example, the emission spectra of Cream 0 after 6 hours exposure compared to the control cream, are shown in **Figure 6****.**

### Example 2 - Absorption/emission spectra of biophotonic cream 'F' before and after illumination

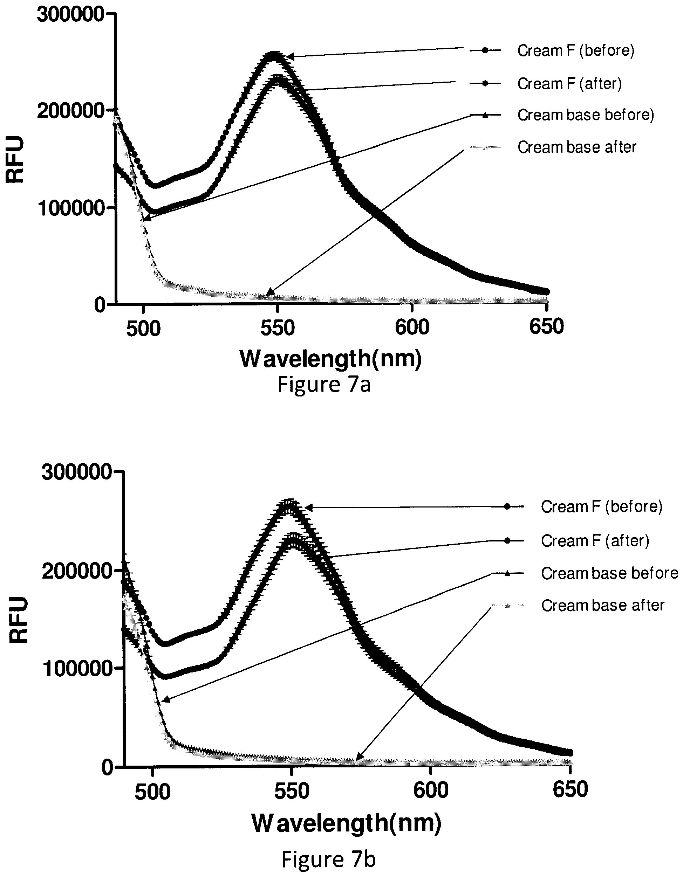

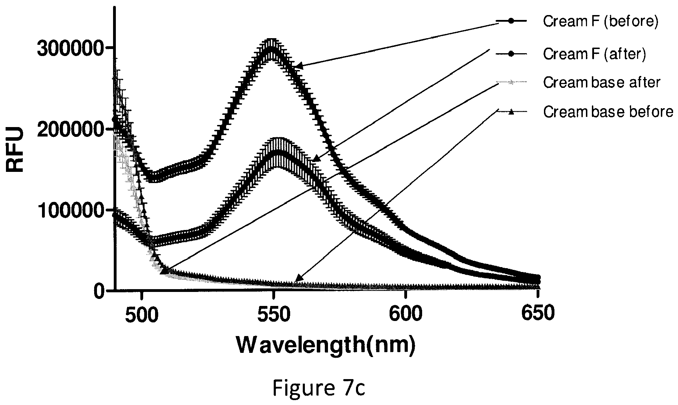

Another composition ('Cream F'), according to an embodiment of the present disclosure, was evaluated for biophotonic activity. Cream F comprised 0.001% chromophore content in a base cream including healing and other active agents. Two mm of the composition was applied to a plastic plate and left on a laboratory bench under the same ceiling lights as Example 1 for about 3, 6 and 16.5 hours. **Figures 7A, 7B** and **7C** show the emission spectra (measured in the same manner as Example 1) of the Cream F composition and a control cream (base cream alone) left under the light for about 3, 6 and 16.5 hours, compared to the Cream F composition and the base cream control at time zero. The fluorescence emitted by the Cream F composition after light exposure was lower than that seen at time zero. This reduction in fluorescence indicates a reduction in biophotonic activity which suggests that the composition was photoactivated by the light. The same pattern of reduced fluorescence was seen when the composition was left under the desk lamp of Example 1 for 3, 6 and 16.5 hours. When activated by a blue light, the composition photobleached much faster with a higher rate of emitted fluorescence compared with the white light results of Figures 7A, 7B and 7C.

### Example 3 - Absorption/emission spectra of Fluorescein and Eosin Y in a gel

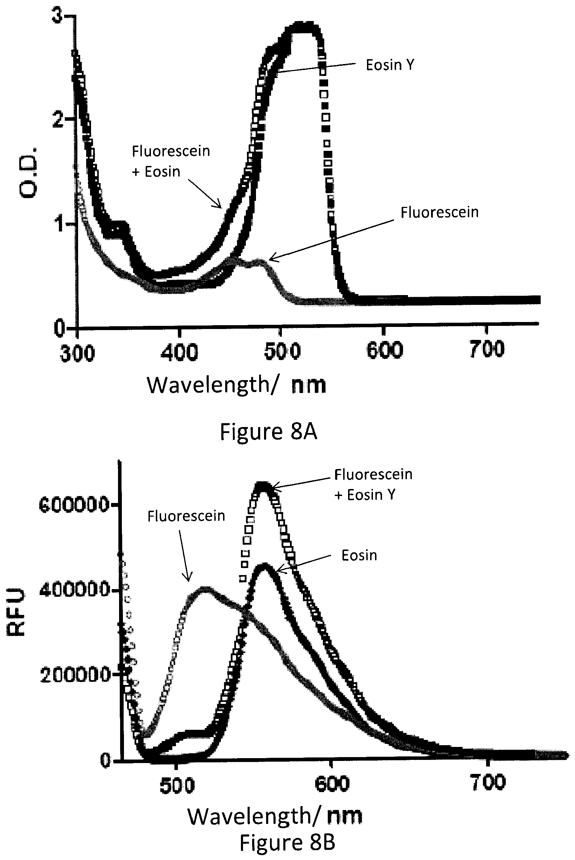

The photodynamic properties of (i) Fluorescein sodium salt at about 0.09 mg/mL, (ii) Eosin Y at about 0.305 mg/mL, and (iii) a mixture of Fluorescein sodium salt at about 0.09 mg/mL and Eosin Y at about 0.305 mg/mL, all in a gel (12% carbamide) were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.The absorption and emission spectra are shown in **Figures 8A** and **8B** which indicate an energy transfer between the chromophores in the combination. It is to be reasonably inferred that this energy transfer can also occur in biophotonic compositions of the present disclosure. In particular a broader absorption and emission spectra was achieved with the Eosin Y and Fluorescein chromophore combination, compared with the individual chromophores. This means that the multiple chromophore composition can be activated with a broader bandwidth of light, and that the multiple chromophore light can emit a broader bandwidth of light after illumination. In other words, emission from the multi-chromophore composition occurred in a broader range of wavelengths compared to the individual chromophores. In this example, the composition emitted light in the green, yellow and orange wavelengths of the visible spectra. Photobleaching of Eosin Y was observed during illumination.

### Example 4 - Absorption/emission spectra of Fluorescein and Eosin Y in an aqueous solution

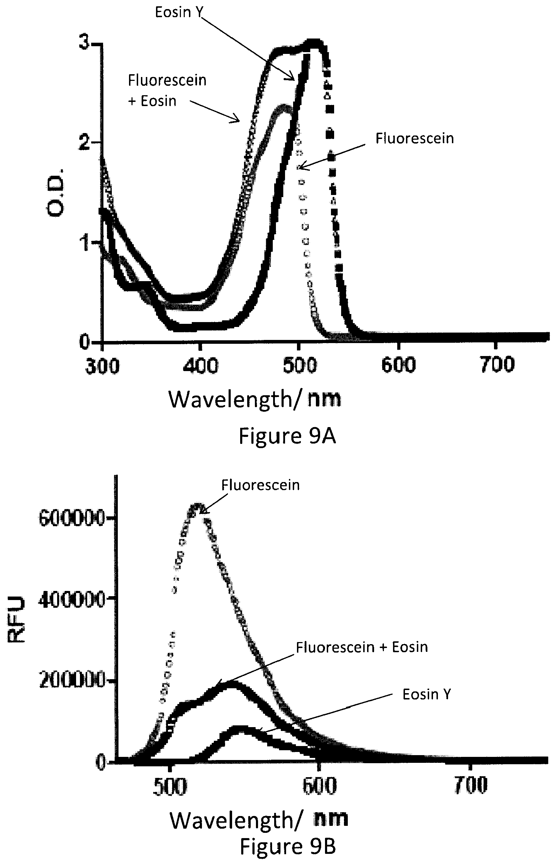

The photodynamic properties of (i) Fluorescein sodium salt at 0.18 mg/mL final concentration, (ii) Eosin Y at about 0.305 mg/mL, and (iii) a mixture of Fluorescein sodium salt at about 0.18 mg/mL and Eosin Y at about 0.305 mg/mL in an aqueous solution were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.The absorption and emission spectra are shown in **Figures 9A** and **9B** which indicate an energy transfer between the chromophores in the combination. It is to be reasonably inferred that this energy transfer can also occur in biophotonic compositions of the present disclosure. Also, as with Figures 8A and 8B, a broader emission spectra was achieved with the Eosin Y and Fluorescein chromophore combination, compared with the individual chromophores. The composition emitted light in the green, yellow and orange wavelengths of the visible spectra. The difference in the absorption and emission spectra between Examples 3 and 4 may be explained by the optical difference in the media (gel in Example 3 and aqueous solution in this example) as well as possibly the effect of doubling the fluorescein concentration. It can be seen that adding Fluorescein to Eosin Y, broadens the bandwidth of the absorption and emission peaks of Eosin Y. This confers on the multiple chromophore combination, the ability to absorb a broader range of wavelengths for photoactivation and to emit a wider range of wavelengths which may confer different therapeutic effects at the same time. Photobleaching of Eosin Y was observed during illumination. Furthermore, results (not shown) indicate that the presence of peroxide in the gel does not affect the absorbance and emission spectra. Peroxide is optional in compositions and methods of the present disclosure.

### Example 5 - Absorption/emission spectra of Phloxine B and Eosin Y in a gel

The photodynamic properties of (i) Phloxine B at 0.25mg/mL final concentration, (ii) Eosin Y at about 0.05 mg/mL, and (iii) a mixture of Phloxine B (0.25mg/mL) and Eosin Y (0.05 mg/mL), all in a 12% carbamide gel were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.

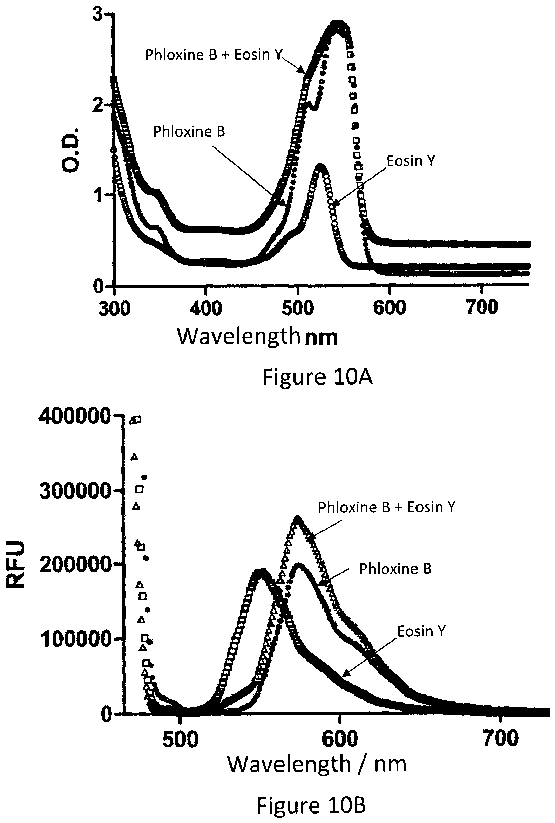

The absorption and emission spectra are shown in **Figures 10A** and **10B** which indicate an energy transfer between the chromophores in the combination. As before, broader absorption and emission spectra were achieved with the Phloxine B and Eosin Y chromophore combination, compared with the individual chromophores. The composition emitted light in the green, yellow, orange and red wavelengths of the visible spectra.

### Example 6 - Absorption/emission spectra of an aqueous solution of Phloxine B and Eosin Y

The photodynamic properties of (i) Phloxine B at 0.25mg/mL final concentration, (ii) Eosin Y at about 0.08 mg/mL, and (iii) a mixture of Phloxine B (0.25mg/mL) and Eosin Y (0.08 mg/mL), all in an aqueous solution were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.

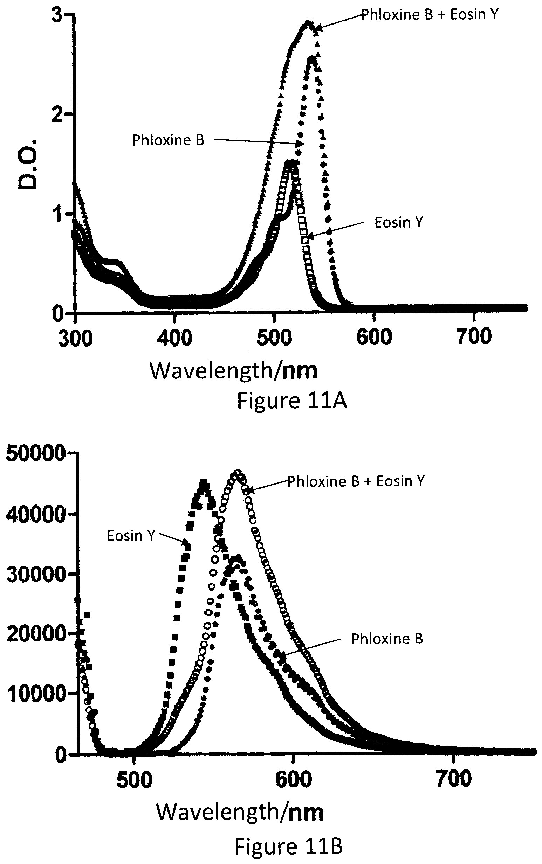

The absorption and emission spectra are shown in **Figures 11A** and **11B** which indicate an energy transfer between the chromophores in the combination. Broader absorption and emission spectra were achieved with the Phloxine B and Eosin Y chromophore combination, compared with the individual chromophores. The composition emitted light in the green, yellow, orange and red wavelengths of the visible spectra.

### Example 7 - Absorption/emission spectra of Rose Bengal and Eosin Y in a gel

The photodynamic properties of (i) Eosin Y at 0.305 mg/mL final concentration, (ii) Rose Bengal at about 0.085 mg/mL, and (iii) a mixture of Eosin Y (0.305mg/mL) and Rose Bengal (0.085 mg/mL), all in a 12% carbamide gel were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.

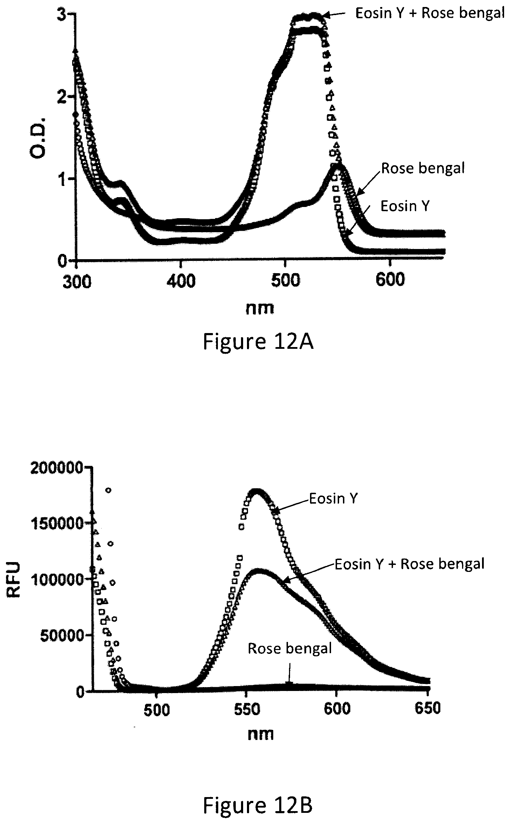

The absorption and emission spectra are shown in **Figures 12A** and **12B** which indicate an energy transfer between the chromophores in the combination. For this particular combination of chromophores and at this concentration, a higher absorption was achieved with the chromophore combination, compared with the individual chromophores. The emission spectra of this specific combination had a lower power density than for Eosin Y alone. In the absence of a temperature rise in the composition during or after illumination, this apparent loss of energy may be attributed to reactive Oxygen species generation.

### Example 8 - Absorption/emission spectra of Fluorescein and Phloxine B in a gel

The photodynamic properties of (i) Fluorescein at about 100µg/g final concentration, (ii) Phloxine B at about 100µg/g, and (iii) a mixture of Fluorescein (100µg/g) and Phloxine B (100µg/g), all in a 12% carbamide gel were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.

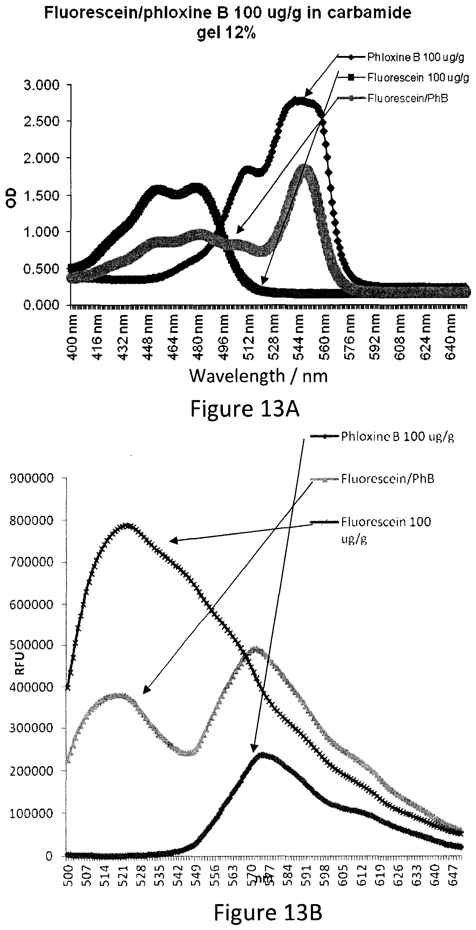

The absorption and emission spectra are shown in **Figures 13A** and **13B** which indicate an energy transfer between the chromophores in the combination. For this particular combination of chromophores and at this concentration, for the chromophore combination two peaks corresponding to fluorescein and phloxine B emission was absorved, with a higher peak at around 577 nm absorption, compared with the individual chromophores.

### Example 9 - Absorption/emission spectra of Fluorescein and Rose Bengal in a gel

The photodynamic properties of (i) Fluorescein at about 100µg/g final concentration, (ii) Rose Bengal at about 100µg/g, and (iii) a mixture of Fluorescein (100µg/g) and Phloxine B (100µg/g), all in a 12% carbamide gel were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.

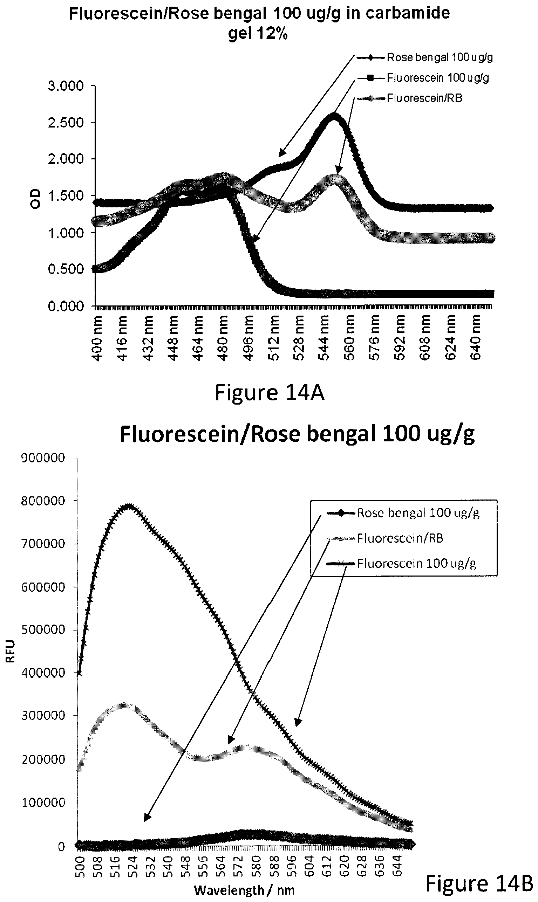

The absorption and emission spectra are shown in **Figures 14A** and **14B** which indicate an energy transfer between the chromophores in the combination. For this particular combination of chromophores and at this concentration, two emission peaks were observed in the combined chromophore composition with the combined composition having a higher peak at around 580 nm, compared with the individual chromophores.

### Example 10 - Absorption/emission spectra of Eosin Y, Fluorescein and Rose Bengal in a gel

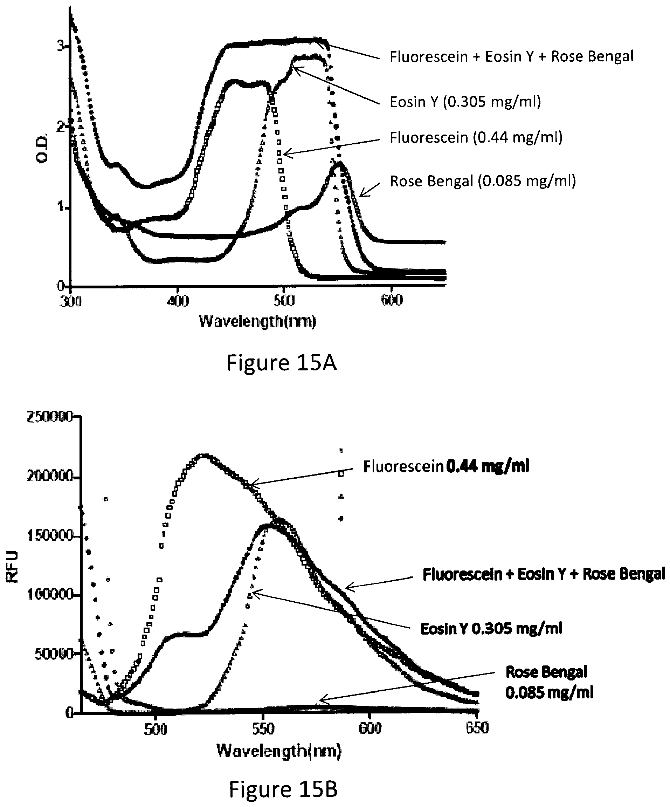

The photodynamic properties of (i) Rose Bengal at about 0.085 mg/mL, (ii) Fluorescein sodium salt at about 0.44 mg/mL final concentration, (ii) Eosin Y at about 0.305 mg/mL, and (iii) a mixture of (i), (ii) and (iii) in a gel were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.The absorbance and emission spectra are shown in **Figures 15A** and **15B** which indicate an energy transfer between the chromophores in the chromophore combination.

### Example 11 - Absorption/emission spectra of Eosin Y, Fluorescein and Rose Bengal in an aqueous solution

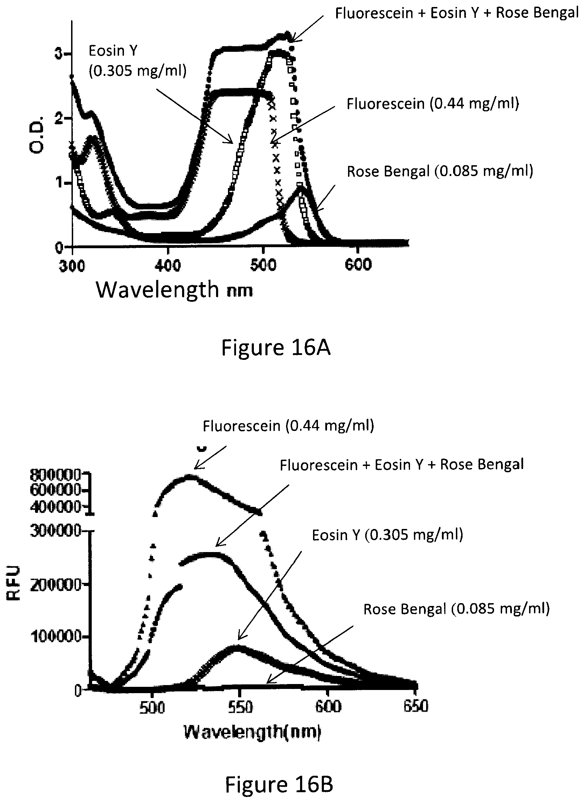

The photodynamic properties of (i) Rose Bengal at about 0.085 mg/mL, (ii) Fluorescein sodium salt at about 0.44 mg/mL final concentration, (ii) Eosin Y at about 0.305 mg/mL, and (iii) a mixture of (i), (ii) and (iii) in an aqueous solution (Set A), were evaluated. A flexstation 384 II spectrophotometer was used to measure emitted fluorescence with the following parameters: mode fluorescence, excitation 460 nm, emission spectra 465-750 nm. The absorbance was read using a synergy HT microplate reader: mode absorbance; spectra between 300-650nm.The absorbance and emission spectra are shown in **Figures 16A** and **16B** which indicate an energy transfer between the chromophores in the chromophore combination.

In reference to the absorption and emission spectra of the compositions of the present disclosure within a carbamide peroxide gel, the same spectra was obtained for the same chromophores in a gel without the peroxide.

### Example 12 - Eosin Y and Fluorescein induce collagen formation

A composition according to an embodiment of the present invention, comprising 0.01% Eosin Y and 0.01% Fluorescein in a carrier matrix (1.8% carbopol gel) was evaluated for its potential to induce collagen formation. Dermal human fibroblasts were plated in glass-bottomed dishes with wells (MatTek®). There were approximately 4000 cells per well. After 48 hours, the glass-bottomed dishes were inverted and the cells were treated through the glass bottom with (i) a no light (control), (ii) sunlight exposure for about 13 minutes at noon (control), (iii) the composition applied to the glass well bottom on the other side of the cells (no light exposure), (iv) the composition applied to the glass well bottom on the other side of the cells (sun light exposure for about 13 minutes at noon), and (v) the composition applied to the glass well bottom on the other side of the cells (blue light exposure for about 5 minutes). In the case of (iii), (iv) and (v), there was no direct contact between the cells and the composition. In the case of (iv) and (v), the cells were exposed to emitted light from and through the Eosin Y and Fluorescein composition when exposed to sunlight and blue light respectively. An at least partial photobleaching was observed in (iv) and (v). After the treatment, the cells were washed and incubated in regular medium for 48 hours. A collagen assay was then performed on the supernatant using the Picro-Sirius red method. This involved adding Sirius red dye solution in picric acid to the supernatant, incubating with gentle agitation for 30 minutes followed by centrifugation to form a pellet. The pellet was washed first with 0.1N HCl and then 0.5 N NaOH to remove free dye. After centrifugation, the suspension was read at 540 nm for collagen type I. The results are shown in Table 1.

**Table 1 - A qualitative comparison of collagen type I concentration in a dermal human fibroblast supernatant exposed to (i) a no light (control), (ii) sunlight exposure for about 13 minutes at noon (control), (iii) any light emitted from a Eosin Y and Fluorescein composition through a glass separation (no light exposure), (iv) any light emitted from a Eosin Y and Fluorescein composition through a glass separation (sun light exposure for about 13 minutes at noon), and (v) the composition applied to the glass well bottom on the other side of the cells (blue light exposure for about 5 minutes). ++ indicates collagen levels about twice as high as +, and +++ indicates collagen levels about three times as high as +.**

| | No light (control) | Sunlight (control) | Eosin Y + Fluorescein - no light | Eosin and Fluorescein - sunlight | Eosin and Fluorescein - blue light |
|---|---|---|---|---|---|
| Collagen concentration | + | + | ++ | +++ | +++ |

There was a statistical difference between the collagen levels induced by the Eosin Y and Fluorescein composition exposed to sunlight and blue light compared to the no light and sunlight alone controls.
Collagen generation is indicative of a potential for tissue repair including stabilization of granulation tissue and decreasing of wound size. It is also linked to reduction of fine lines, a decrease in pore size, improvement of texture and improvement of tensile strength of intact skin. The emission spectra of the Eosin Y and Fluorescein composition of this example had a single peak emission with a wavelength that ranged from about 480-620 nm. Following illumination with sunlight, the power density of the peak was reduced indicating an at least partial photobleaching in 13 minutes, which was also observed by a change in colour of the composition. The rate of fluorescence emission/photobleaching was slower when illuminated by sunlight (white light) compared to Eosin Y and Fluorescein compositions (e.g. compositions of Examples 5 and 6) when activated by blue light.

The relationship between the power density of light received by the tissues with illuminating time was investigated for a number of compositions. It was found that the power density of the activating light was low initially and increased with time. This correlates with the light absorbing chromophores photobleaching and more of the activating light passing through the composition to reach tissues. In parallel, the fluorescent light emitted by the composition decreased with time as one or more of the chromophores photobleached. Overall, the total power density of the light treating the tissues increased gradually over illumination time.

It should be appreciated that the invention is not limited to the particular embodiments described and illustrated herein but includes all modifications and variations falling within the scope of the invention as defined in the appended claims.

The present disclosure discloses at least the following items:
Item 1. A method for rejuvenating skin or maintaining skin condition comprising:
   - topically applying a composition onto a skin area, the composition comprising a first fluorescent chromophore, a second fluorescent chromophore, and a dermatologically acceptable carrier, wherein the first and second chromophores are capable of absorbing light in the visible range of the electromagnetic spectrum and have a total concentration by weight in the composition of about 0.00 lwt% to less than about 0.5wt%; and
   - illuminating said composition with ambient light and/or direct light containing a wavelength that can be absorbed by the first fluorescent chromophore.
Item 2. The method of item 1, wherein at least one of the first and second fluorescent chromophores are present in a concentration from about 0.00 lwt% to less than about 0.1 wt% per weight of the total composition.
Item 3. The method of item 1, wherein at least one of the first and second fluorescent chromophores are present in a concentration from about 0.001 wt% to less than about 0.01wt% per weight of the total composition.
Item 4. The method of any of items 1-3, wherein the first and second fluorescent chromophores absorb light having a wavelength in the range of about 380-800 nm, about 380-700 nm, about 380 to 600 nm, about 400-500 nm, about 450-650 nm, about 600-700 nm, or about 650-750 nm.
Item 5. The method of any of items 1-4, wherein the first or the second fluorescent chromophore has an emission spectrum that overlaps at least 20% of an absorption spectrum of the other of the first and second chromophores.
Item 6. The method of any of items 1-5, wherein photoactivation of one of the first and the second fluorescent chromophores can photoactivate the other of the first and second fluorescent chromophores.
Item 7. The method of any of items 1-6, wherein one of the first and second chromophores absorbs at an average peak wavelength that is relatively longer than that of the other of the first and second chromophores, and within the range of about 10-100 nm.
Item 8. The method of any of items 1-7, wherein at least one of the first and second fluorescent chromophores is in a soluble form in the composition.
Item 9. The method of any of items 1-8, wherein the composition can emit light from about 480 nm to about 650 nm when exposed to the light.
Item 10. The method of any of items 1-9, wherein the composition can emit light from about 500 nm to about 650 nm when exposed to the light.
Item 11. The method of any of items 1-10, wherein the composition can emit light from about 520 nm to about 650 nm when exposed to the light.
Item 12. The method of any of items 1-11, wherein the composition can emit light from about 520 nm to about 600 nm when exposed to the light.
Item 13. The method of any of items 1-12, wherein the first fluorescent chromophore is a xanthene dye.
Item 14. The method of any of items 1-13, wherein the second chromophore is a chlorophyll dye, such as chlorophyllin, chlorophyll a or chlorophyll b.
Item 15. The method of any of items 1-12, wherein the first and the second fluorescent dye are xanthene dyes.
Item 16. The method of item 13 or item 15, wherein the xanthene dye is selected from fluorescein, eosin Y, erythrosine B, eosin B, Phloxine B, rose bengal, and derivatives thereof
Item 17. The method of any of items 1-16, wherein the dermatologically acceptable carrier is optically transmissive to at least one of an absorption and an emission wavelengths of the first fluorescent chromophore.
Item 18. The method of any of items 1-17, wherein the dermatologically acceptable carrier is aqueous.
Item 19. The method of any of items 1-18, wherein the dermatologically acceptable carrier is an emulsion.
Item 20. The method of any of items 1-18, wherein the dermatologically acceptable carrier is a skin cream, lotion or serum.
Item 21. The method of any of items 1-20, wherein the first and/or the second fluorescent chromophore undergoes at least partial photobleaching upon exposure to the light.
Item 22. The method of any of items 1-21, wherein the composition does not include cleansers.
Item 23. The method of any of items 1-22, wherein the composition does not include a self-tanning agent.
Item 24. The method of any of items 1-23, wherein the composition does not include a non-fluorescent colourant.
Item 25. The method of any of items 1-24, wherein illuminating the composition with the light causes an increase in collagen synthesis in the tissue onto which the composition is topically applied.
Item 26. The method of any of items 1-25, wherein illuminating the composition with the light has a luminosity increasing effect on the tissue onto which the composition is topically applied.
Item 27. The method of any of items 1-26, wherein illuminating the composition with the light from the visible portion of the electromagnetic spectrum is not accompanied by concomitant generation of heat on the tissue to which the composition is topically applied.
Item 28. The method of any of items 1-27, wherein the source of the ambient light or the direct light is the sun.
Item 29. The method of any of items 1-27, wherein the source of the direct light is a mobile device having an emission spectra which overlaps an absorption spectra of at least one of the first and second fluorescent chromophores.
Item 30. The method of any of items 1-27, wherein the source of the direct light is a display screen of a television or a computer having an emission spectra which overlaps an absorption spectra of at least one of the first and second fluorescent chromophores.
Item 31. The method of any of items 1-30, wherein the composition is topically applied to the skin daily.
Item 32. The method of any of items 1-31, wherein the composition is illuminated for about 15 minutes, about 30 minutes, about 45 minutes, about 1 hour, about 2 hours, about 3 hours, about 4 hours, about 5 hours, about 6 hours, about 7 hours or about 8 hours.
Item 33. The method of any of items 1-31, wherein the composition is illuminated intermittently.
Item 34. A cosmetic composition for topical cosmetic use, the composition comprising a first fluorescent chromophore, a second fluorescent chromophore, and a dermatologically acceptable carrier, wherein the first and second chromophores are capable of absorbing light in the visible range of the electromagnetic spectrum and have a total concentration by weight in the composition of about 0.001wt to less than about 0.5wt%.
Item 35. The composition of item 34, wherein at least one of the first and second fluorescent chromophores are present in a concentration from about 0.001 to less than about 0.1% per weight of the total composition.
Item 36. The composition of item 34, wherein at least one of the first and second fluorescent chromophores are present in a concentration from about 0.00 lwt% to less than about 0.01wt% per weight of the total composition.
Item 37. The composition of any of items 34-36, wherein the first and second fluorescent dyes absorb light having a wavelength in the range of about 380-800 nm, about 380-700 nm, about 380 to 600 nm, about 400-500 nm, about 450-650 nm, about 600-700 nm, or about 650-750 nm.
Item 38. The composition of any of items 34-37, wherein the first or the second fluorescent chromophore has an emission spectrum that overlaps at least 20% of an absorption spectrum of the other of the first and second chromophores.
Item 39. The composition of any of items 34-38, wherein photoactivation of one of the first or the second fluorescent chromophores can photoactivate the other of the first and second fluorescent chromophores.
Item 40. The composition of any of items 34-39, wherein one of the first and second chromophores absorbs at an average peak wavelength that is relatively longer than that of the other of the first and second chromophores, and within the range of about 10-100 nm.
Item 41. The composition of any of items 34-40, wherein at least one of the first and second fluorescent chromophores is in a soluble form in the composition.
Item 42. The composition of any of items 34-41 , wherein the composition can emit light having a wavelength from about 480 nm to about 650 nm when exposed to the ambient and/or direct light.
Item 43. The composition of any of items 34-42, herein the composition can emit light having a wavelength from about 500 nm to about 650 nm when exposed to the ambient and/or direct light.
Item 44. The composition of any of items 34-43, herein the composition can emit light having a wavelength from about 520 nm to about 650 nm when exposed to the ambient and/or direct light.
Item 45. The composition of any of items 34-44, herein the composition can emit light having a wavelength from about 520 nm to about 600 nm when exposed to the ambient and/or direct light.
Item 46. The composition of any of items 34-45, wherein the first fluorescent chromophore is a xanthene dye.
Item 47. The composition of any of items 34-46, wherein the second chromophore is a chlorophyll dye, such as chlorophyllin, chlorophyll a or chlorophyll b.
Item 48. The composition of any of items 34-45, wherein the first and the second fluorescent dye are xanthene dyes.
Item 49. The composition of any of items 48, wherein the xanthene dyes are selected from fluorescein, eosin Y, erythrosine B, eosin B, Phloxine B, rose bengal, and derivatives thereof.
Item 50. The composition of any of items 34-49, wherein the dermatologically acceptable carrier is optically transmissive to at least one of an absorption and an emission wavelengths of the first fluorescent chromophore.
Item 51. The composition of any of items 34-50, wherein the dermatologically acceptable carrier is aqueous.
Item 52. The composition of any of items 34-51, wherein the dermatologically acceptable carrier is an emulsion.
Item 53. The composition of any of items 34-51 , wherein the dermatologically acceptable carrier is a skin cream, lotion or serum.
Item 54. The composition of any of items 34-53, wherein the first and/or the second fluorescent chromophore undergoes at least partial photobleaching upon exposure to the light.
Item 55. The composition of any of items 34-54, wherein the composition does not include cleansers.
Item 56. The composition of any of items 34-55, wherein the composition does not include a self-tanning agent.
Item 57. The composition of any of items 34-56, wherein the composition does not include a non-fluorescent colourant.
Item 58. The composition of any of items 34-57, wherein exposure of the composition with light causes an increase in collagen synthesis in the tissue onto which the composition is topically applied.
Item 59. The composition of any of items 34-58, wherein exposure of the composition with light has a luminosity increasing effect on tissue onto which the composition is topically applied.
Item 60. The composition of any of items 34-59, wherein exposure of the composition with light is not accompanied by concomitant generation of heat on the tissue to which the composition is topically applied.
Item 61. A composition for topical cosmetic use, the composition comprising: a first fluorescent chromophore and a dermatologically acceptable carrier; wherein the first chromophore is capable of absorbing and/or emitting light in the visible range of the electromagnetic spectrum and wherein the composition does not substantially visibly colour the skin after topical application of the composition to the skin in use.
Item 62. The composition of item 61, wherein the concentration of the first fluorescent chromophore is such that photoactivation of the chromophore by ambient light and/or direct sunlight does not cause the chromophore to emit fluorescent light substantially visible to the human eye.
Item 63. The composition of item 61 or 62, herein the first fluorescent chromophore is present in a concentration less than about 0.5% per weight of the total concentration.
Item 64. The composition of any of items 61-63, wherein the first fluorescent chromophore is present in a concentration less than about 0.1% per weight of the total composition.
Item 65. The composition of any of items 61-63, wherein the first fluorescent chromophore is present in a concentration from about 0.001% to less than about 0.5% per weight of the total composition.
Item 66. The composition of any of items 61-63, wherein the first fluorescent chromophore is present in a concentration from about 0.001% to less than about 0.1% per weight of the total composition.
Item 67. The composition of any of items 61-63, wherein the first fluorescent chromophore is present in a concentration from about 0.001 % to less than about 0.01% per weight of the total composition.
Item 68. The composition of any of items 61-63, wherein the first fluorescent chromophore is present in a concentration from about 0.001% to less than about 0.005% per weight of the total composition.
Item 69. The composition of any of items 61-68, wherein the first fluorescent dye absorbs light having a wavelength in the range of about 380-800 nm, about 380-700 nm, about 380 to 600 nm, about 400-500 nm, about 450-650 nm, about 600-700 nm, or about 650-750 nm.
Item 70. The composition of any of items 61-69, wherein the first fluorescent chromophore is in a soluble form in the composition.
Item 71. The composition of any of items 61 -70, wherein the first fluorescent chromophore is a xanthene dye.
Item 72. The composition of any of items 61-70, wherein the first fluorescent dye is selected from fluorescein, eosin Y, erythrosine B, eosin B, Phloxine B, and rose bengal.
Item 73. The composition of any of items 61-70, further comprising a second fluorescent chromophore.
Item 74. The composition of item 73, wherein second wherein the first or the second fluorescent chromophore has an emission spectrum that overlaps at least 20% of an absorption spectrum of the other of the first and second chromophores.
Item 75. The composition of item 73 or 74, wherein photoactivation of one of the first or the second chromophores can photoactivate the other of the first and second chromophores.
Item 76. The composition of any of items 73-75, wherein one of the first and second chromophores absorbs at an average peak wavelength that is relatively longer than that of the other of the first and second chromophores, and within the range of about 10-100 nm.
Item 77. The composition of any of items 74-76, wherein second fluorescent chromophore is a xanthene dye.
Item 78. The composition of item 74-77, wherein the second fluorescent dye is selected from fluorescein, eosin Y, erythrosine B, eosin B, Phloxine B, and rose bengal.
Item 79. The composition of any of items 74-78, wherein the second chromophore is a chlorophyll dye, selected from chlorophyllin, chlorophyll a and chlorophyll b.
Item 80. The composition of any of items 74-79, wherein the dermatologically acceptable carrier is optically transmissive to at least one of an absorption and an emission wavelengths of the first fluorescent chromophore.
Item 81. The composition of any of items 62-80, wherein the dermatologically acceptable carrier is aqueous.
Item 82. The composition of any of items 62-81, wherein the dermatologically acceptable carrier is an emulsion.
Item 83. The composition of any of items 62-81, wherein the dermatologically acceptable carrier is a skin cream, lotion or serum.
Item 84. The composition of any of items 62-83, wherein the first fluorescent chromophore at least partially photobleaches upon exposure to ambient light or sunlight.
Item 85. The composition of any of items 62-84, wherein the composition does not include cleansers.
Item 86. The composition of any of items 62-85, wherein the composition does not include a self-tanning agent.
Item 87. The composition of any of items 62-86, wherein the composition does not include a non-fluorescent colourant.
Item 88. The composition of any of items 62-87, wherein exposure of the composition with the light causes an increase in collagen synthesis in the tissue onto which the composition is topically applied.
Item 89. The composition of any of items 62-88, wherein exposure of the composition with the light has a luminosity increasing effect on tissue onto which the composition is topically applied.
Item 90. The composition of any of items 62-89, wherein exposure of the composition with the light is not accompanied by concomitant generation of heat on the tissue to which the composition is topically applied.
Item 91. A method for rejuvenating skin or maintaining skin condition comprising:
   - topically applying a composition onto a skin area, the composition comprising a first fluorescent chromophore and a dermatologically acceptable carrier, wherein the first fluorescent chromophore is capable of absorbing light in the visible range of the electromagnetic spectrum; and
   - illuminating said composition with ambient light and/or direct light containing a wavelength that can be absorbed by the first fluorescent chromophore; wherein the composition does not substantially visibly colour the skin after topical application of the composition to the skin in use.
Item 92. A method for stimulating collagen formation comprising:
   - topically applying a composition onto a skin area, the composition comprising a first fluorescent chromophore and a dermatologically acceptable carrier, wherein the first fluorescent chromophore is capable of absorbing light in the visible range of the electromagnetic spectrum; and
   - illuminating said composition with ambient light and/or direct light containing a wavelength that can be absorbed by the first fluorescent chromophore; wherein the composition does not substantially visibly colour the skin after topical application of the composition to the skin in use. 93. A method for rejuvenating skin or maintaining skin condition comprising:
   - topically applying a composition onto a skin area, the composition comprising a first fluorescent chromophore, a second fluorescent chromophore, and a dermatologically acceptable carrier, wherein the first and second chromophores are capable of absorbing light in the visible range of the electromagnetic spectrum; and
   - illuminating said composition with ambient light and/or direct light containing a wavelength that can be absorbed by the first fluorescent chromophore, wherein the composition does not substantially visibly colour the skin after topical application of the composition to the skin in use.
Item 94. The method of any of items 91-93, wherein the concentration of the first fluorescent chromophore is such that photoactivation of the chromophore by ambient light and/or direct sunlight does not cause the chromophore to emit fluorescent light substantially visible to the human eye.
Item 95. The method of any of items 91-94, wherein the first fluorescent chromophore is present in a concentration less than about 0.5 wt per weight of the total concentration.
Item 96. The method of any of items 91-94, wherein the first fluorescent chromophore is present in a concentration less than about 0.1 wt% per weight of the total composition.
Item 97. The method of any of items 91-94, wherein the first fluorescent chromophore is present in a concentration from about 0.001 wt% to less than about 0.5 wt% per weight of the total composition.
Item 98. The method of any of items 91 -94, wherein the first fluorescent chromophore is present in a concentration from about 0.001 wt to less than about 0.1 wt% per weight of the total composition.
Item 99. The method of any of items 91-94, wherein the first fluorescent chromophore is present in a concentration from about 0.001 wt% to less than about 0.01 wt per weight of the total composition.
Item 100. The method of any of items 91 -99, wherein the first fluorescent chromophore absorbs light having a wavelength in the range of about 380-800 nm, about 380-700 nm, about 380 to 600 nm, about 400-500 nm, about 450-650 nm, about 600-700 nm, or about 650-750 nm.
Item 101. The method of any of items 91-100, wherein the first fluorescent chromophore is in a soluble form in the composition.
Item 102. The method of any of items 91-101, wherein the composition can emit light having a wavelength of about 480 nm to about 650 nm when illuminated by the ambient and/or direct light.
Item 103. The method of any of items 91-101, wherein the composition can emit light having a wavelength of from about 500 nm to about 650 nm when illuminated by the ambient and/or direct light.
Item 104. The method of any of items 91-101, wherein the composition can emit light having a wavelength of from about 520 nm to about 650 nm when illuminated by the ambient and/or direct light.
Item 105. The method of any of items 91-101, wherein the composition can emit light having a wavelength of from about 520 nm to about 600 nm when illuminated by the ambient and/or direct light.
Item 106. The method of any of items 91-105, wherein the first fluorescent chromophore is a xanthene dye.
Item 107. The method of item 106, wherein the xanthene dye is selected from fluorescein, eosin Y, erythrosine B, eosin B, Phloxine B, rose bengal, and derivatives thereof.
Item 108. The method of any of items 91-107, wherein the dermatologically acceptable carrier is optically transmissive to at least one of an absorption and an emission wavelengths of the first fluorescent chromophore.
Item 109. The method of any of items 91-108, wherein the dermatologically acceptable carrier is aqueous.
Item 110. The method of any of items 91-109, wherein the dermatologically acceptable carrier is an emulsion.
Item 111. The method of any of items 91-110, wherein the dermatologically acceptable carrier is a skin cream, lotion or serum.
Item 112. The method of any of items 91-111, wherein the first fluorescent chromophore undergoes at least partial photobleaching when illuminated with the ambient and/or direct light.
Item 113. The method of any of items 91-112, wherein the composition does not include cleansers.
Item 114. The method of any of items 91-113, wherein the composition does not include a self-tanning agent.
Item 115. The method of any of items 91-114, wherein the composition does not include a non-fluorescent colourant.
Item 116. The method of any of items 91-115, wherein illumination of the composition causes an increase in collagen synthesis in the tissue onto which the composition is topically applied.
Item 117. The method of any of items 91- 116, wherein illumination of the composition has a luminosity increasing effect on tissue onto which the composition is topically applied.
Item 118. The method of any of items 91-117, wherein illumination of the composition is not accompanied by concomitant generation of heat on the tissue to which the composition is topically applied.
Item 119. The method of any of items 91-118, wherein the source of the direct or the ambient light is the sun.
Item 120. The method of any of items 91-119, wherein the source of the direct light is a mobile device having an emission spectra which overlaps an absorption spectra of at least one of the first and second fluorescent chromophores.
Item 121. The method of any of items 91-118, wherein the source of the light is a display screen of a television or a computer having an emission spectra which overlaps an absorption spectra of at least one of the first and second fluorescent chromophores.
Item 122. The method of any of items 91-121, wherein the composition is topically applied to the skin daily.
Item 123. The method of any of items 91-122, wherein the composition is illuminated for about 15 minutes, about 30 minutes, about 45 minutes, about 1 hour, about 2 hours, about 3 hours, about 4 hours, about 5 hours, about 6 hours, about 7 hours or about 8 hours.
Item 124. Use of a composition of any of items 34-60, or items 61-90 for skin rejuvenation or skin condition maintenance.
Item 125. The use of item 124, further for use in stimulating collagen formation in the skin.
Item 126. Use of a composition for rejuvenating skin or maintaining skin condition, the composition comprising:
   a first fluorescent chromophore,
   a second fluorescent chromophore, and
   a dermatologically acceptable carrier,
   wherein the first and second chromophores are capable of absorbing light in the visible range of the electromagnetic spectrum and have a total concentration by weight in the composition of about 0.001wt% to less than about 0.5wt; and
   wherein said composition is suitable for illumination with ambient light and/or direct light containing a wavelength that can be absorbed by the first fluorescent chromophore.
Item 127. A method for rejuvenating skin, maintaining skin condition and/or for stimulating/increasing collagen formation comprising illuminating skin using an overlap in an emission spectra of a first and second fluorescent chromophore to emit light having a wavelength of about 480 nm to about 650 nm.
Item 128. A method of using a cascade of energy transfer between at least a first and a second fluorescent chromophore to absorb and/or emit light within the visible range of the electromagnetic spectrum for rejuvenating skin or maintaining skin condition.

## Claims

1. A cosmetic composition for topical use, the composition comprising a first fluorescent chromophore, a second fluorescent chromophore, and a dermatologically acceptable carrier, wherein the first and second chromophores are capable of absorbing light in the visible range of the electromagnetic spectrum and have a total concentration by weight in the composition of about 0.001 wt% to less than about 0.5 wt%.

2. The cosmetic composition of claim 1, wherein the first and second fluorescent chromophores are present in a concentration from about 0.001% to less than about 0.1% per weight of the total composition.

3. The cosmetic composition of claim 1 or 2, wherein the first or the second fluorescent chromophore has an emission spectrum that overlaps at least about 20% of an absorption spectrum of the other of the first and second chromophores.

4. The cosmetic composition of any one of claims 1 to 3, wherein photoactivation of the first or the second fluorescent chromophores can photoactivate the second or the first fluorescent chromophore.

5. The cosmetic composition of any one of claims 1 to 4, wherein the first and second chromophores absorbs at an average peak wavelength that is relatively longer than that of the other of the second or the first chromophore, and within the range of between about 10 nm and 100 nm.

6. The cosmetic composition of any one of claims 1 to 5, wherein the composition can emit light having a wavelength from between about 480 nm to about 650 nm when exposed to ambient, actinic, and/or direct light.

7. The cosmetic composition of any one of claims 1 to 6, wherein the first fluorescent chromophore is a xanthene dye.

8. The cosmetic composition of any one of claims 1 to 7, wherein the second chromophore is a chlorophyll dye selected from the group consisting of chlorophyllin, chlorophyll a, and chlorophyll b.

9. The cosmetic composition of any one of claims 1 to 8, wherein the first and the second fluorescent chromophores are xanthene dyes.

10. The cosmetic composition of claim 9, wherein the xanthene dyes are selected from the group consisting of fluorescein, eosin Y, erythrosine B, eosin B, Phloxine B, rose bengal, and derivatives thereof.

11. The cosmetic composition of any one of claims 1 to 10, wherein the dermatologically acceptable carrier is optically transmissive to at least one of an absorption and an emission wavelengths of the first fluorescent chromophore.

12. The cosmetic composition of any one of claims 1 to 11, wherein the first and/or the second fluorescent chromophore undergoes at least partial photobleaching upon exposure to the light.

13. The cosmetic composition of any one of claims 1 to 12, wherein exposure of the composition with light causes an increase in collagen synthesis in the tissue onto which the composition is topically applied.

14. A composition for use in a method for rejuvenating skin or maintaining skin condition, comprising:
a first fluorescent chromophore, a second fluorescent chromophore, and a dermatologically acceptable carrier, wherein the first and second chromophores are capable of absorbing light in the visible range of the electromagnetic spectrum and have a total concentration by weight in the composition of about 0.001 wt% to less than about 0.5 wt%; wherein the composition is suitable for illumination with ambient light and/or direct light containing a wavelength that can be absorbed by the first fluorescent chromophore.

15. The composition of claim 14, wherein the rejuvenating skin or maintaining skin condition comprises the prevention or reduction of facial lines, fine lines, wrinkles, crow's feet, dark eye circles, blemishes, age spots, stretch marks, or combinations thereof.
